(19) Europäisches Patentamt
European Patent Office
Office européen des brevets

(11) **EP 4 172 589 B1**

(12) **EUROPEAN PATENT SPECIFICATION**

(45) Date of publication and mention
of the grant of the patent:
**09.10.2024 Bulletin 2024/41**

(21) Application number: **21739984.9**

(22) Date of filing: **28.06.2021**

(51) International Patent Classification (IPC):
**G01N 1/28** *(2006.01)*  **G01N 33/543** *(2006.01)*
**G01N 33/68** *(2006.01)*  **C12N 7/02** *(2006.01)*
**C12Q 1/68** *(2018.01)*  **C12Q 1/70** *(2006.01)*
**C12Q 1/6806** *(2018.01)*

(52) Cooperative Patent Classification (CPC):
**G01N 1/28; C12N 7/00; G01N 33/5076;
G01N 33/54346;** G01N 2333/70596

(86) International application number:
**PCT/EP2021/067748**

(87) International publication number:
**WO 2021/260231 (30.12.2021 Gazette 2021/52)**

(54) **BIOLOGICAL VESICLES ISOLATION AND DISCOVERY METHODS AND SYSTEMS**

VERFAHREN UND SYSTEME ZUR ISOLIERUNG UND ENTDECKUNG BIOLOGISCHER VESIKEL

PROCÉDÉS ET SYSTÈMES D'ISOLEMENT ET DE DÉCOUVERTE DE VÉSICULES BIOLOGIQUES

(84) Designated Contracting States:
**AL AT BE BG CH CY CZ DE DK EE ES FI FR GR
HR HU IE IS IT LI LT LU LV MC MK MT NL NO PL
PT RO RS SE SI SK SM TR**

(30) Priority: **26.06.2020 GB 202009799**

(43) Date of publication of application:
**03.05.2023 Bulletin 2023/18**

(73) Proprietor: **Mursla Limited
London
W1U 6QX (GB)**

(72) Inventors:
• **DIAS, Tomás Miguel De Freitas
Cambridge CB4 2LE (GB)**
• **ARSÈNE, Pierre
London W1U 6QX (GB)**

(74) Representative: **Marks & Clerk LLP
15 Fetter Lane
London EC4A 1BW (GB)**

(56) References cited:
**US-A1- 2019 025 330**

• **AN YU ET AL: "Magneto-Mediated
Electrochemical Sensor for Simultaneous
Analysis of Breast Cancer Exosomal Proteins",
ANALYTICAL CHEMISTRY, vol. 92, no. 7, 11
March 2020 (2020-03-11), US, pages 5404 - 5410,
XP055779498, ISSN: 0003-2700, Retrieved from
the Internet
<URL:https://pubs.acs.org/doi/pdf/10.1021/acs.a
nalchem.0c00106> DOI:
10.1021/acs.analchem.0c00106**
• **KSENIIA BORIACHEK ET AL: "Quantum
dot-based sensitive detection of disease specific
exosome in serum", ANALYST, vol. 142, no. 12,
23 May 2017 (2017-05-23), UK, pages 2211 - 2219,
XP055499970, ISSN: 0003-2654, DOI:
10.1039/C7AN00672A**
• **DARIO BRAMBILLA ET AL: "Separation of
Extracellular Vesicles by DNA-Directed
Immunocapturing Followed by Enzymatic
Release", 6 May 2020 (2020-05-06), XP055698920,
Retrieved from the Internet
<URL:https://s3-eu-west-1.amazonaws.com/ite
mpdf74155353254prod/12234683/Separation_of
_Extracellular_Vesicles_by_DNA-Directed_Imm
unocapturing_Followed_by_Enzymatic_Release
_v1.pdf> DOI: 10.26434/chemrxiv.12234683.v1**

## Description

## FIELD OF INVENTION

The present invention generally relates to a method and system of isolating a target biological entity from a biofluid, with the use of functionalised capture particles.

## BACKGROUND

Extracellular vesicles (EVs) are released by all cells, including prokaryotes and eukaryotes, during normal physiological processes. Two types of EVs abundant in organisms include Ectosomes and Exosomes, which are distinguished in that Exosomes are released on the exocytosis of Multivesicular bodies (MVBs), whereas Ectosomes are assembled and released from the plasma membrane of a cell. In other words, Exosomes are necessarily endosomal in origin. Exosomes range in size from around 40 to 160 nm in diameter, or even as small as 20 nm, and Ectosomes range from around 50 nm to around 1 $\mu$m in diameter.

Although the pathways of Exosome formation and their physiological role is not generally understood and thus an active area of research, it is generally understood that EVs, including Exosomes, may contain any number of constituents of a cell from which the EV or Exosome originated. In this way, during their biogenesis, Exosomes may assimilate a variety of cellular content specific to the cell of origin. Constituents of EVs/Exosomes may include DNA, RNA, lipids, cell-surface proteins, and proteins dissolved in the cytosol of the cell (cytosolic proteins).

EVs are also involved in many pathophysiological processes, such as immune responses, inflammation, tumour growth and infection. Furthermore, the nucleic acid and protein content from a healthy versus a diseased patient differ, meaning that EVs are a valuable biomarker for many diseased states.

Furthermore, a number of other biological entities such as viruses have remarkably similar structures with EVs - sharing features such as diameter, density, structure, cargo type, cargo origin and cell entry and exit mechanisms, despite having different functions.

Viruses in particular are a significant cause of morbidity and mortality in human populations. Given the health and economic costs associated with viral infections efficiently detecting the presence of viral pathogens in a clinical sample is of vital importance.

There therefore remains a need in the state-of-the-art to reliably detect, isolate, quantify, and characterise, the presence and nature of such biological entities of interest for both research and diagnostic applications.

Known documents include: US 2019/0025330 A; Analytical Chemistry (Apr 2020) Vol 92, pp 5404-5410, "Magneto-mediated electrochemical sensor...", An et al; and The Analyst (2017), Vol 142, pp 2211-2219, "Quantum dot-based sensitive detection...", Boriachek et al.

US 2019/025330 A1 discloses a method of purifying a population of nanoscale extracellular vesicles, said method comprising: providing a triorthogonal linker comprising an antibody that is attached by said linker to a magnetic bead where said linker comprise a photocleavable core disposed between the antibody and the magnetic bead and where said antibody is an antibody that binds a marker displayed on an nsEV; contacting a biological sample comprising nsEVs with said triorthogonal linker under conditions where said antibody binds to an nsEV in said sample to form a complex comprising a magnetic bead attached by said linker to an nsEV; utilizing a magnet/magnetic field to isolate said complex from other components in said sample; exposing said complex to light to cleave said photocleavable core and separate the magnetic bead from the antibody-bound nsEV; and utilizing a magnet/magnetic field to separate said magnetic bead from the antibody-bound nsEV to provide a substantially purified population of antibody-bound nsEvs.

## SUMMARY OF THE INVENTION

According to claim 1 there is provided a method of isolating target extracellular vesicles, EVs, from a biofluid comprising a plurality of target EVs and non-target EVs. The method comprises: introducing a plurality of EV capture microparticles to the biofluid to obtain a precursor mixture, wherein the plurality of EV capture microparticles are functionalised, via a plurality of first linkers, with EV-specific binding agents specific to an EV surface marker, such that a plurality of bound microparticle-EV assemblies is formed in the precursor mixture; extracting the bound microparticle-EV assemblies to obtain a cleaned precursor mixture; introducing a plurality of target capture nanoparticles to obtain an assembly mixture, wherein the plurality of target capture nanoparticles are functionalised with target-specific binding agents receptive to surface markers comprised on the target EVs, such that the target capture nanoparticles bind to the plurality of target EV; and cleaving the plurality of first linkers to dissociate at least the plurality of target EVs from the EV capture microparticles. After the cleaving, the method further comprises extracting the plurality of EV capture microparticles and applying at least one of dielectrophoresis or centrifugation to extract the plurality of target capture nanoparticles, such that the target EVs are separated from the non-target EVs.

It will be appreciated that, after the final separation step (of target from non-target EVs) dielectrophoresis or centrifugation, the target EVs may still be bound to the target capture nanoparticles.

The target capture nanoparticles can be introduced either to the initial precursor mixture or to the cleaned precursor mixture; irrespective of the order, a clean solution free of contaminants is still formed as a result of the capture of EVs by the EV capture microparticles. It should be appreciated that the optional step of applying dielectrophoresis comprises the application of an alternating electric field, which acts upon the target capture nanoparticles. Thus, in examples, a dielectrophoretic force can be induced on the target capture nanoparticles such that the nanoparticles (and any target particles to which they are bound) are drawn towards, or repelled from, the origin of the alternating electrical field.

The method may further comprise, removing unbound target capture nanoparticles before the cleaving of the plurality of first linkers. In detail, after introducing the plurality of target capture nanoparticles, assemblies comprising EV capture microparticles, target capture nanoparticles, non-target EVs, and target EVs, are formed. However, some target capture nanoparticles may not have bound/captured to any target EVs (for example, if an excess of target capture nanoparticles is used.) Therefore, in some examples, any unbound target capture nanoparticles are desired to be removed before the step of cleaving the microparticles from the EVs. Removal/extraction of target capture nanoparticles prior to the cleaving step is advantageous in examples where bound target capture nanoparticles are subsequently used as the detection or quantification labels.

For example, the removal of the unbound target capture nanoparticles may comprise sequestering the above-mentioned assemblies and washing away any unbound target-capture nanoparticles. For example, in examples where the EV capture microparticles are magnetic, the assemblies comprising the microparticles (or microparticles) may be sequestered by a magnet, thus allowing free particles (including the unbound target capture nanoparticles) to be washed away.

The plurality of target capture nanoparticles are functionalised with the target-specific binding agents, via a plurality of second linkers that are not cleaved during the step of cleaving the plurality of first linkers. Accordingly, the method further comprises: after separating the plurality of target EVs from the non-target EVs, cleaving the plurality of second linkers to dissociate the plurality of target capture nanoparticles from the plurality of target EVs; and applying at least one of dielectrophoresis or centrifugation to extract the dissociated target capture nanoparticles and isolate a plurality of unbound target EVs. Isolated target EVs are thus available for downstream applications such as biomarker discovery, and the dissociated target capture nanoparticles may be counted separately in order to determine the precise number of specifically isolated EVs (as, beneficially, target capture nanoparticles become bound to target EVs or viruses in a 1:1 ratio, meaning that the number of nanoparticles precisely indicates the number of isolated EVs).

Therefore, it will be appreciated that, in some examples, the first and second linkers are responsive to different cleaving treatments, and cannot be cleaved using the same treatment. Specifically, the plurality of second linkers may comprise disulfide bonds, which are cleaved by treatment with a reducing or alkaline reagent. The plurality of first linkers may be photo-cleavable linkers, which are cleaved by exposure to UV or near-UV light. Beneficially, UV light does not cleave the disulfide bonds comprised in the second linkers.

The plurality of first linkers may alternatively comprise DNA or RNA, which are cleaved by treatment with an enzyme. For example, the enzyme may be a nuclease. At least one of the EV-specific binding agents used in any of the above examples, and the target-specific binding agent, may be an antibody. For example, the antibody may be receptive to antigens comprised on a surface of the EVs or the target EVs.

In an alternate example, the plurality of first linkers comprise DNA or RNA, and the plurality of target capture nanoparticles comprise a plurality of second linkers comprising DNA or RNA, where the method may further comprise: treating the assembly mixture with an enzyme to cleave a plurality of bound first linkers and a plurality of bound second linkers, the plurality of bound first and second linkers being bound to a target EV, to dissociate the plurality of target EVs from each of the EV capture microparticles and the target capture nanoparticles, such that the plurality of target EVs are separated from the plurality of non-target EVs. Advantageously, only a single cleaving step is required here, such that non-target EVs remain bound to the microparticles and can easily be extracted away from the target EVs. Furthermore, the plurality of second linkers may be terminated with two receptors: a first receptor bearing target-specific antibodies that are receptive to target-specific markers comprised on the target EVs; and a second receptor bearing additional DNA or RNA which is receptive to the DNA or RNA in the plurality of first linkers. In this example, the method may further comprise applying at least one of dielectrophoresis, or centrifugation, to extract the dissociated target capture nanoparticles, to isolate a plurality of unbound target EVs.

Generally, the above examples of the isolation method may comprise: introducing the plurality of unbound target EVs to a second plurality of the EV capture microparticles; introducing a second plurality of target capture nanoparticles functionalised with second target-specific binding agents receptive to surface markers comprised on a subset of the unbound target EVs; cleaving the plurality of first linkers on the second plurality of the EV capture microparticles and extracting said second plurality of EV capture microparticles; and applying at least one of dielectrophoresis or centrifugation to extract the second plurality of target capture nanoparticles and isolate the subset of the target EVs. Furthermore, a second cleavage step may again be performed in order to sever the linkers on the second plurality of target capture nanoparticles, in order to dissociate the subset of target EVs from the nanoparticles.

Advantageously, the use of a second plurality of the EV capture microparticles and a second plurality of target capture nanoparticles enables a further selection of EVs, so that a refined sub-population of EVs can be isolated. This is enabled by virtue of the fact that the isolation method keeps the EVs intact, such that an isolated population of EVs can be carried forward for a further round of isolation. In principle, multiple, even indefinite, rounds of isolation can be performed using increasingly specific target-markers on the target capture nanoparticles in order to obtain an ultra-refined set of target EVs.

The plurality of EV capture microparticles may be magnetic, wherein the steps of extracting the bound microparticle-EV assemblies and extracting the plurality of EV capture microparticles comprise applying a magnetic field.

The method may further comprise characterising the plurality of target EVs, the characterising comprising one or more of: polymerase chain reaction (PCR), mass spectrometry, DNA or RNA sequencing, and liquid chromatography. It may be advantageous to combine some of the above methods, for example, performing liquid chromatography and mass spectrometry in tandem. The plurality of unbound target EVs may be lysed prior to the characterisation, to release an internal EV content, such that the characterisation is performed on the internal EV content. In one embodiment, the internal EV content may be characterised to identify one or more disease-specific biomarkers. In a further example, it is has been found that EVs from virus-infected cells, such as SARS-CoV2 and other retroviruses, contain viral proteins and RNA cargo. Accordingly, in a further embodiment, the internal EV content may be characterised to identify the presence of viral proteins and/or RNA cargo. As such, the present method may enable the detection of a virus and/or the diagnosis of a viral infection by characterising EV content as described above.

In some examples, once the target EVs have been isolated from the non-target EVs, a step is performed to quantify the number of target particles present. Accordingly, each of the target EVs of the plurality of target EVs, having been separated from the non-target EVs, may be comprised in a bound EV-nanoparticle assembly, where the method further comprises: applying an electric field between a pair of electrodes to concentrate the bound EV-nanoparticle assembly in a sensing region, wherein the sensing region is defined by a region between the pair of electrodes, which are separated by a lateral distance of less than 100 nm; applying a nanoparticle sensing voltage between the electrodes; characterizing a response of the sensing region to the nanoparticle sensing voltage to determine EV quantification data; and quantifying a number of target EVs from the quantification data.

Preferably, a length dimension of the plurality of EV capture microparticles is about an order of magnitude greater (i.e. around ten times greater) than a length dimension of each of the target EVs, or about two orders of magnitude greater (i.e. around one hundred times greater) in terms of surface area, such that, in the plurality of bound microparticle-EV assemblies, no two target EVs are proximate when bound to a surface of the plurality of EV capture microparticles.

The meaning of proximate will be understood to be a relative term, and generally means that target EVs are spread out over the surface of the, larger, microparticle. This has the result that all target EVs are separated, over the surface of the microparticles, by at least their own diameter. In preferable examples, the EVs are separated by at least the size dimension of the target capture nanoparticles such that it is impossible for one target capture nanoparticle to intercept/capture more than one target particles.

Each target capture nanoparticle of the plurality of target capture nanoparticles thus preferably captures at most one target EV. In more detail, in preferred examples, the result of relative sizes of the nanoparticles to the microparticles is a ratio of exactly 1:1 of target EVs to nanoparticles in the bound target-nanoparticles assemblies. This provides advantages for quantification purposes, i.e., such that it can be reliably inferred that the number of nanoparticles sensed equates exactly to the number of target EVs present.

The plurality of target EVs may have a size dimension of around 20 nm to 160 nm, and the plurality of target EVs may be exosomes. Furthermore, all the EVs in a sample biofluid may be exosomes. In other words, the biofluid may contain only or substantially exosomes, wherein the target EVs are a sub-population of the exosomes in the biofluid.

In this example, the sub-population may contain a surface marker representative of a particular disease and/or a particular organ or tissue of an organism. In other words, the exosome carries a cell-surface specific biomarker.

According to another aspect of the present disclosure, there is provided a method, not falling under the scope of the appended claims, of isolating target exosomes from a biofluid comprising a plurality of target exosomes, a plurality of non-target exosomes, and other extracellular vesicles, EVs. The method comprises: introducing a plurality of EV capture microparticles to the biofluid to obtain a precursor mixture, wherein the plurality of EV capture microparticles are functionalised, via a plurality of first linkers, with one or more EV-specific binding agents specific to an EV surface marker, such that a plurality of bound microparticle-EV assemblies is formed in the precursor mixture; extracting the bound microparticle-EV assemblies to obtain a cleaned precursor mixture; introducing a plurality of exosome capture nanoparticles, wherein the plurality of exosome capture nanoparticles are functionalised, via a plurality of second linkers, with one or more exosome-specific binding agents receptive to exosome surface markers, such that the exosome capture nanoparticles bind to the plurality of non-target exosomes and a plurality of target exosomes; and cleaving the plurality of first linkers to dissociate the plurality of EVs from the EV capture microparticles. After the cleaving, the method further comprises extracting the plurality of EV capture microparticles and applying at least one of dielectrophoresis or centrifugation to extract the plurality of exosome capture nanoparticles bound to the pluralities of non-target exosomes and target exosomes; and cleaving the plurality of second linkers to dissociate the plurality of exosome capture nanoparticles, and applying at least one of dielectrophoresis or centrifugation to extract the dissociated exosome capture nanoparticles. The method further comprises introducing a second plurality of the EV or exosome capture microparticles and introducing a plurality of target-exosome capture nanoparticles functionalised, via the second linkers, with target-specific binding agents receptive to surface markers comprised on the plurality of target exosomes; cleaving the plurality of first linkers on the second plurality of the EV capture microparticles and extracting said second plurality of EV capture microparticles; applying at least one of dielectrophoresis or centrifugation to extract the plurality of target-exosome capture nanoparticles bound to the plurality of target exosomes; and cleaving the plurality of second linkers to dissociate the plurality of target-exosome capture nanoparticles, and applying at least one of dielectrophoresis or centrifugation to extract the dissociated target-exosome capture nanoparticles and isolate the plurality of target exosomes.

In general, this aspect comprises a first isolation step where a population of exosomes are isolated from a biofluid, and a second step where a subset of the initially-isolated exosomes are isolated using a second set of target-capture nanoparticles. The target capture nanoparticles may be gold nanoparticles, functionalised with one or more specific binding agents that target a highly specific (i.e., disease or tissue specific) exosome surface marker. For example, the first isolation step may comprise isolating all exosomes. The second step may comprise isolating a target exosome from the exosome population, e.g. all exosomes carrying a target-specific marker. The target-specific marker may be a biomarker or a tissue-specific biomarker, e.g., from the isolated brain-originating exosomes, all exosomes carrying a disease-specific marker which express a particular protein, merely for example, Amyloid Beta.

Accordingly, in a further aspect of the disclosure, there is provided a method, not falling under the scope of the appended claims, for diagnosing a neurodegenerative disorder, the method comprising an exosome marker selection step, a neuronal marker selection step and a disease marker selection step. In one example, the method comprises the following steps:

Exosome markers selection:

(a) introducing a plurality of EV capture microparticles to a biofluid obtained from a patient to obtain a precursor mixture, wherein the plurality of EV capture microparticles are functionalised, via a plurality of first linkers, with one or more EV-specific binding agent specific to an EV surface marker, such that a plurality of bound microparticle-EV assemblies is formed in the precursor mixture, wherein preferably the EV-surface marker is an exosome-specific marker, such as a tetraspanin, such as CD9, CD63, CD81, CD326, CD82, CD37 or CD41;

(b) extracting the bound microparticle-EV assemblies to obtain a cleaned precursor mixture;

Neuronal markers selection:

(c) introducing a plurality of first target-capture nanoparticles, wherein the plurality of target-capture nanoparticles are functionalised, via a plurality of second linkers, with one or more target-specific binding agents, wherein the target is a brain-specific biomarker, such that the target capture nanoparticles bind to the plurality of target exosomes;

(d) cleaving the plurality of first linkers to dissociate the plurality of EVs from the EV capture microparticles;

(e) extracting the plurality of EV capture microparticles and applying at least one of dielectrophoresis or centrifugation to extract the plurality of target-capture nanoparticles bound to the target EV;

(f) optionally removing unbound target-capture nanoparticles; and

(g) cleaving the plurality of second linkers to dissociate the plurality of target-capture nanoparticles, and applying dielectrophoresis or centrifugation to extract the dissociated target-capture nanoparticles to obtain only EVs expressing the target biomarker;

wherein the method further comprises either

(h) lysing the target EVs and analysing the lysed contents for the presence of amyloid-beta; or

Disease markers selection:

(i) introducing a plurality of second-target capture nanoparticles functionalised via a plurality of third linkers with a binding agent capable of specific for a neurodegenerative disease biomarker; applying at least one of dielectrophoresis or centrifugation to extract the plurality of second target-capture nanoparticles bound to the biomarker-expressing EVs; and cleaving the plurality of third linkers to dissociate the plurality of second target-capture nanoparticles, and applying at least one of dielectrophoresis or centrifugation to extract the dissociated target-capture nanoparticles and isolate the plurality of EVs that express the disease biomarker.

In one example, the neurodegenerative disease biomarker is selected from Amyloid beta (Aβ42), amyloid precursor protein (APP), alpha-synuclein, close homolog of L1, insulin receptor substrate 1 (IRS-1), neural cell adhesion molecule (NCAM) and tau protein, where the presence of the biomarker in or on the surface of the EV is indicative of a neurodegenerative disorder.

It will be understood that the plurality of EVs released from the EV capture microparticles, after the first cleaving of the plurality of first linkers, includes all exosomes (both target and non-target) which are bound to the exosomes capture microparticles and other (including exosomes not ultimately targeted by the nanoparticles).

Thus, this aspect has the benefit of being able to isolate a highly specific subset of exosomes from a complex fluid containing a variety of EVs, where a proportion of the EVs are non-target exosomes and target exosomes. The complex biofluid may further contain contaminants such as peptides, protein aggregates, cell fragments, cholesterol lipoproteins, and the like. In particular, the biofluid may contain free-floating (i.e., not EV-bound) biomarkers or EV-bound biomarkers from organs/tissues other than the targeted organs/tissues, which are disregarded by the selection of tissue-specific markers (e.g. neuronal markers selection).

The plurality of EV capture microparticles may be magnetic, and wherein the steps of extracting the bound microparticle-EV assemblies and extracting the plurality of EV capture microparticles comprise applying a magnetic field.

Generally, the biofluid may be obtained from a cell culture, or directly from a patient. For example, the sample may be any suitable body fluid, such as for example blood, urine, saliva, sputum or lymph cerebrospinal fluid.

According to yet another aspect of the invention, there is provided a method of detecting the presence of a disease biomarker in a biofluid obtained from a sample from a patient, the method comprising isolating a plurality of unbound target exosomes bearing the disease biomarker using the steps of any of claims 1 to 12.

In some embodiments, where the disease biomarker is contained in the exosome, the method may further comprise the step of lysing the target exosome and analysing the lysed contents for the presence of the biomarker.

In a further aspect of the disclosure, there is provided a method, not falling under the scope of the appended claims, of diagnosing and/or treating a disease, the method comprising detecting the presence of one or more disease biomarkers from a biofluid as described above, wherein the presence of the disease-specific biomarker on or in the exosome is indicative of disease. The method may further comprise the step of administering a treatment to a patient diagnosed with the disease.

According to any of the above aspects and examples, the EV surface markers, which are receptive to the EV-specific binding agent, may be a transmembrane protein, preferably a tetraspanin, such as a tetraspanin selected from at least one of CD9, CD63, CD81, CD326, CD82, CD37 or CD41. In one embodiment, the EV-capture microparticles may be functionalised with one or more EV-specific binding agents, and preferably two or three different EV-specific binding agents, wherein the binding agents bind to a EV-specific transmembrane protein, such as a tetraspanin preferably selected from CD9, CD63, CD81, CD326, CD82, CD37 and CD41. In one example, the EV-capture microparticles are functionalised with three different EV-specific binding agents, wherein the binding agents bind to CD63, CD81 and CD9.

The surface markers comprised on the target EVs may be disease-specific or tissue specific markers, and the target-specific binding agents are configured to bind with a disease-specific or a tissue-specific marker, and not with a generic EV marker. Again, in one example, the target-specific nanoparticles may be functionalised with one or more different disease or tissue-specific markers. This may be particularly useful to enhance the specificity or sensitivity of the method, especially where one biomarker is expressed at a low concentration. In one example, the target-specific nanoparticles may be functionalised with two or three different disease or target-specific markers.

Preferably, the target capture nanoparticles are gold nanoparticles. However, the target capture nanoparticles may also comprise silver, or generally any other conductive, e.g. metallic or semi-metallic, material. A size dimension of target capture nanoparticles may be around 50 nm to 300 nm, or preferably around 200 nm. In examples where target EVs or viruses are isolated for discovery purposes, the GNPs may be even smaller than 50 nm.

In general, an extracellular vesicle, EV, is a biological vesicle of around 20 - 160 nm size. Nevertheless, the isolation methods described above may also be carried out, using the equivalent steps, to isolate lipoproteins (i.e. low density lipoproteins and high-density lipoproteins, LDL and HDLs), and other biological entities having a lipid bilayer as a cell-membrane, including viruses.

As such, in a further aspect of the disclosure, there is provided a method, not falling under the scope of the appended claims, of isolating a target virus from a biofluid. The method comprises: introducing a plurality of virus-specific capture microparticles to the biofluid to obtain a precursor mixture, wherein the plurality of virus-specific capture microparticles are functionalised, via a plurality of first linkers, with a first virus-specific binding agent specific to a first viral surface marker, such that a plurality of bound microparticle-viral assemblies is formed in the precursor mixture; extracting the bound microparticle-viral assemblies to obtain a cleaned precursor mixture; introducing a plurality of viral capture nanoparticles to obtain an assembly mixture, wherein the plurality of viral capture nanoparticles are functionalised with a second virus-specific binding agent receptive to a second viral surface marker comprised on the target virus, such that the viral capture nanoparticles bind to the plurality of target viruses; and cleaving the plurality of first linkers to dissociate at least the plurality of target viruses from the viral capture microparticles. After the cleaving, the method further comprises extracting the plurality of viral capture microparticles and applying at least one of dielectrophoresis or centrifugation to extract the plurality of viral capture nanoparticles, such that the target viruses are isolated from the biofluid.

In this example, the viral surface markers may be any marker present on the viral envelope, such as viral glycoproteins, as for example Spike proteins. Preferably, the first and second viral surface markers are from the same virus and as such the use of specific markers can be used to identify that a given virus is present in a sample. Examples of viral glycoproteins include gp160 (present in the HIV virus), hemagglutinin, neuraminidase and the M2 protein from influenza, and the E glycoprotein from SARS-CoV-2. Alternatively, the viral surface marker may be a Spike protein such as the S glycoprotein from SARS-CoV-2. Other examples would be known to the skilled person. The present method has the advantage that by having a first and second step both specific for viral-specific surface markers from the same virus (or strain) the specificity of the method to detect a given virus in a sample is increased.

In one example, the method described above may be particularly valuable in vaccine production (and in particular for attenuated viruses) as a quality control measure. In another example, the method can be used to determine the presence of a target virus in a biofluid, such as a patient sample. While a virus can be selected using viral-specific binding agents as described above, in some examples the isolated virus may be further analysed, for example, using known techniques such as PCR or RT-PCR to identify the virus. Accordingly, in a further aspect of the disclosure, there is provided a method, not falling under the scope of the appended claims, of diagnosing a viral infection in a patient, the method comprising isolating the virus from the biofluid using the above described method. In a yet further aspect, there is provided a method, not falling under the scope of the appended claims, of treating a viral infection, the method comprising diagnosing the viral infection and administering a suitable treatment to a patient in need thereof.

Additional examples

According to another aspect, methods as described above may be for isolating target extracellular vesicles, EVs, or virions (e.g. viruses). Generally, although in some scenarios virions may be seen as a type of EV, in other examples, the above-disclosed method for isolating target extracellular vesicles may optionally relate only to isolation of EVs and exclude the isolation of virions.

In accordance with the above-described methods for isolating target extracellular vesicles, the method may further comprise, after separating the plurality of target EVs from the non-target EVs, introducing an acidic or oxidising reagent to dissociate the plurality of target capture nanoparticles from the respective plurality of target EVs. The acidic or oxidising reagent may dissociate, or catalyse the dissolution of, the target-specific binding agent comprised on a surface of the target capture nanoparticles. Thus, a mixture containing separated target capture nanoparticles and target EVs may be obtained. It is thus possible to further apply at least one of dielectrophoresis or centrifugation to extract the dissociated target capture nanoparticles and isolate a plurality of unbound target EVs.

In this example, advantageously, no (cleavable) linker molecule is necessary for connecting the target capture nanoparticle with the target-specific binding agent (although it will be appreciated that the capture nanoparticle is still linked with the binding agent). The acidic or oxidising reagent acts directly on the target-specific binding agent (which may be an antibody, or the like) to sever the bond or association between the target capture nanoparticle and the surface markers comprised on the surface of the target EV. For example, where the target-specific binding agent is an antibody, it may not be necessary to modify the antibody with a (cleavable) linker, as a reagent having an acidic pH can be used to catalyze the dissolution of the antibody bound to the surface markers comprised on the target EVs. One example of such an arrangement is gold nanoparticle(GNP)-Streptavidin linked directly to Biotin-antibodies. Generally, said surface markers comprised on the target EVs may be referred to as epitopes.

Regarding the target capture nanoparticles, which may comprise metals such as silver or gold and the like, target capture nanoparticles may generally be made of, or comprise, other conductive and/or metallic and/or semi-metallic material. Yet further, in some examples, target capture nanoparticles may comprise other non-conductive, e.g. non-metallic, and/or polymeric material.

Furthermore, as mentioned above in respect of the method of detecting the presence of a disease biomarker, it is possible to lyse the plurality of unbound target exosomes to produce lysed contents, e.g. to obtain cytosolic proteins that may subsequently be characterised in the ways described herein. In other examples, however, it is possible to report cytosolic contents of EVs without destroying/lysing the EV. For example, permeabilization may be performed with a suitable surfactant, such as like Saponin, Triton X-100 and others. The surfactant, when used, can form complexes with cholesterol and/or other lipids in the EV membrane (or in cell membranes) to generate pores, thus leaving internal content available for reporting via generally any of the above-described methods. Thus, target capture nanoparticles may be used as described above to report on the presence of specific cytosolic markers/proteins. This has the advantage that cytosolic proteins of target EVs can be detected whilst keeping target EVs intact, as lysis is not required to detect said cytosolic proteins. Therefore, intact target EVs may be retained for further downstream applications.

According to a related aspect, there is provided a method of isolating target extracellular vesicles, EVs, from a biofluid comprising a plurality of target EVs and non-target EVs, the method comprising: introducing a plurality of EV capture microparticles to the biofluid to obtain a precursor mixture, wherein the plurality of EV capture microparticles are functionalised, via a plurality of first linkers, with EV-specific binding agents specific to an EV surface marker, such that a plurality of bound microparticle-EV assemblies is formed in the precursor mixture; extracting the bound microparticle-EV assemblies to obtain a cleaned precursor mixture; introducing a plurality of target capture nanoparticles to obtain an assembly mixture, wherein the plurality of target capture nanoparticles are functionalised, via a plurality of second linkers, with target-specific binding agents receptive to surface markers comprised on the target EVs, such that the target capture nanoparticles bind to the plurality of target EVs; cleaving the plurality of first linkers to dissociate at least the plurality of target EVs from the EV capture microparticles, wherein the plurality of second linkers are not cleaved; after the cleaving, extracting the plurality of EV capture microparticles and applying at least one of dielectrophoresis or centrifugation to extract the plurality of target capture nanoparticles, such that the target EVs are separated from the non-target EVs; after separating the plurality of target EVs from the non-target EVs, cleaving the plurality of second linkers to dissociate the plurality of target capture nanoparticles from the plurality of target EVs; and applying at least one of dielectrophoresis or centrifugation to extract the dissociated target capture nanoparticles and isolate a plurality of unbound target EVs.

**BRIEF DESCRIPTION OF THE DRAWINGS**

Figs. 1a- 1e show part of an example exosome isolation strategy using photo-cleavable linkages and disulfide linkages;

Figs. 2a - 2e show part of an example exosome isolation strategy using enzyme-cleavable linkages and disulfide linkages;

Figs. 3a-3e show part of an example exosome isolation strategy using RNA/DNA hybrid molecular beacons;

Figs. 4a - 4c show the individual nanoparticles and exosomes present in the isolation strategies illustrated in Figs. 1 - 3;

Figs. 5a and 5b each show scanning electron microscope images of magnetic beads with highlights showing positions of bound target exosomes and nanoparticles;

Fig. 6 illustrates the general structure of an exosome;

Fig. 7 illustrates an apparatus suitable for extracting nanoparticles bound to target molecules using dielectrophoresis;

Fig. 8 illustrates a profile cross-sectional view of an electrode pair with a nano-gap, and an electrical double layer in the surrounding fluid;

Fig. 9 illustrates a method of using dielectrophoresis (DEP) and current measurements to capture and detect a target particle using metal nanoparticles;

Fig. 10 shows a workflow for the process of isolating a target population of particles from a biofluid;

Fig. 11 shows a general workflow for methods of detecting and/or characterising isolated targets; and

Fig. 12 illustrates a model system for carrying out the method steps of Figure 10.

**DETAILED DESCRIPTION**

The inventors have identified a method to isolate sub-populations of extracellular vesicles (EVs) or viruses from biofluids or lab-based cell cultures. The EVs or viruses, when isolated, can be screened in applications such as biomarker discovery for identification of diseases or the identification of novel markers indicative of a particular disease. Furthermore, the presently disclosed method enables identification of new therapy targets, and for the identification of EVs with regenerative ability (e.g. Mesenchymal stem cell EVs).

Extracellular vesicle (EV) is a broad definition and generally includes Exosomes, Ectosomes and Microvesicles, amongst other biomolecules. EVs share the common characteristic of cell membranes formed of a lipid-bilayers, and they lack the ability to replicate. Commonly, within the family of EVs, exosomes are viewed as promising targets for the aforementioned discovery applications. Exosomes are membranous nanometer-sized vesicles (i.e. 40 - 160 nm) that are actively released by all known cells. During their biogenesis, they assimilate a variety of cellular content specific to their parent cells. Exosomes share many membrane-bound proteins at their surface. For example, tetraspanin proteins CD63, CD9 and CD81 are known examples that are commonly targeted as universal exosome biomarkers, present on the surface of exosomes. However, because all tissues are different and the cells that make those tissues express different material, exosomes also contain (typically internally) tissue-specific or cell-specific molecules such as RNAs and proteins. This is also true in diseased cells, where the diseased and aberrant cells may overexpress specific content that can be detected in or on exosomes.

The disclosed method includes at least two steps, which can in general be repeated:

In the first step, EVs, viruses, and/or exosomes are captured on μm-sized particles, preferably through antibody-based affinity, taking advantage of known exosome universal markers (for example, the above mentioned tetraspanins). This first step simultaneously cleans the biofluid from other particles, cell fragments, contaminants and free circulating proteins. The microparticles are preferably magnetic such that they can be extracted using a magnetic field. For example, a specific structure of a suitable microparticle is: a silicon core and covered with a polymer shell composed of highly cross-linked polystyrene; magnetic material (i.e., ferromagnetic material such as iron or nickel) is precipitated in pores distributed throughout the particle. The surface chemistry used to functionalise the microparticles to capturing/sequestering EVs depends on the polymer shell. Generally, the shell is usually hydrophilic which confers an ability to form covalent amide bonds with proteins.

In the second step, sub-populations of target-specific EVs bound to the microparticles used in the first step are captured or tagged with nanoparticles functionalised with target-specific binding agents. These binding agents can be antibodies specific to certain surface markers present only on a subpopulation of EVs. The desired subpopulation of EVs may be exosomes, or a specific family of exosomes, for example. Thus, an assembly of microparticles-EV-nanoparticle is formed. These assemblies can be dissociated such that only the exosomes-nanoparticles complexes are recovered, thus removing from solution the populations of non-target EVs bound to the microparticles.

Generally, this isolation method allows the capture of all EV/exosome populations based on universal markers, in addition to cleaning contaminants from the mixture such as freely circulating proteins, other extracellular vesicles or particles. Next, having captured a general class of EVs, exosomes, or viruses, a sub-population (or sub-class of viruses) of the general class is selected, based on a surface marker indicative of a specific tissue or disease or virus in the second step. This enables accurate characterization of sub-populations of EVs, exosomes, or viruses that would otherwise be difficult or impossible to capture using only one selection/capture step. In particular, having only a target-specific selection step would likely lead to the selection of any target surface marker freely circulating in the biofluid, rather than only on the surface of the EVs or exosomes. Once the target sub-population of EVs or viruses is isolated from non-targets using the above method, the target population is immediately available for analysis in subsequent assays, for example any from the group of: PCR, and in particular RT-PCR, RNA-Seq, ELISA, LC-MS-Chromatography, nanoparticle tracking, and the like. In one example, the EV may be lysed and the protein or nucleic acid content characterised. Such characterisation may involve determining the presence of one or more biomarkers within the EV. Alternatively, such characterisation may lead to the identification of one or more new EV biomarkers.

The nanoparticles are functionalised with more specific binding agents/antibodies than the microparticles. Therefore, the nanoparticles tag only a specific population of EVs bound to the microparticles, which can be retrieved by several methods such as centrifugation or dielectrophoresis and subsequently assessed, where other irrelevant EVs or exosomes are discarded. In this way, purified populations or sub-populations of EVs, exosomes, or viruses bound to the nanoparticles are then dissociated and individually suitable for downstream characterization.

Consistent with the above steps, the micronsized beads select a first population of EVs, exosomes, or viruses. In the second step, nanometre-sized particles select a specific sub-set of the first population. Merely for example, this sub-set may be cancer or other disease-specific exosomes or may be a tissue-specific exosome. These may also be sub-populations of exosomes with regenerative ability or exosomes containing therapeutic value. The micron-sized particles are preferably magnetic beads, of at least 500 nm up to around 5 μm. The microparticles are functionalised with surface-bound binding agents or antibodies, which are attached via cleavable linkers so that the captured particles can later be released. The binding agents are designed to bind with universal markers present on EVs, exosomes, or a particular class of viruses. For example, the microparticles may be functionalised to capture only small EVs (sEVs). The cleavable linkers may be a photo-cleavable linker, a disulfide bridge, DNA or RNA spacers, or DNA-RNA hybrids, and the like (not an exhaustive list).

The nanometre-sized particles are preferably gold nanoparticles (GNPs) of at least 50 nm up to around 300 nm. In some examples, the nanoparticles may be made of materials other than gold. It is generally beneficial for the nanoparticles to be biocompatible, conductive and metallic, however. The nanoparticles are functionalized so that they recognize surface markers only present on a sub-population of EVs or viruses, or a particular sub-class of viruses.

The antibodies are linked to the microparticles through cleavable linkers so that the captured exosomes can later be released. Preferably, the cleavable linkers used to functionalise the nanoparticles are different cleavable linkers to those used on the microparticles, such that cleaving can be performed in two steps. The nanoparticle's cleavable linkers may nevertheless be photocleavable, disulfide bridges, and DNA or RNA spacers cleavable by enzymes, or DNA-RNA hybrids (the list is not exhaustive).

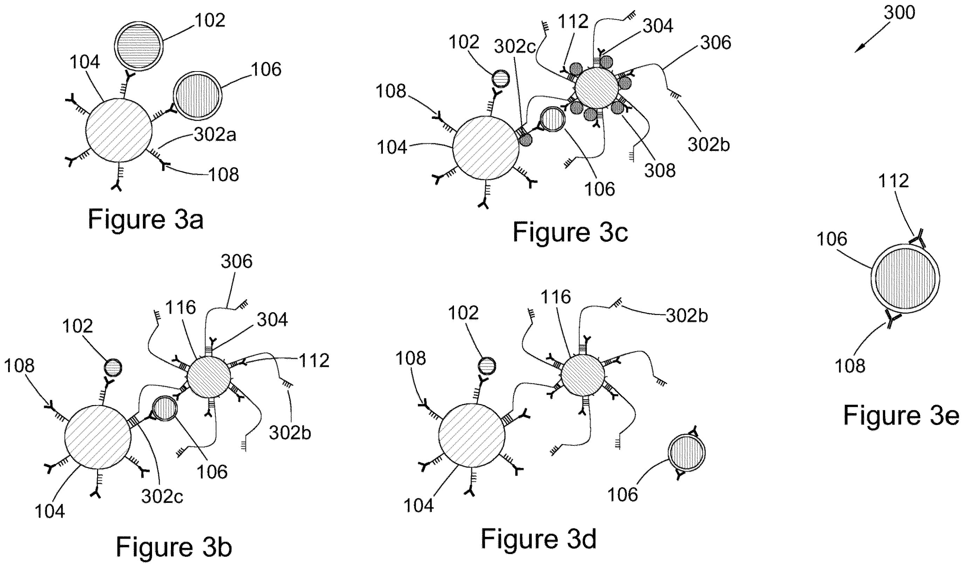

The nanoparticles may, additionally, be prepared with ssDNA, ssRNA or other molecules which, in some embodiments, might facilitate the simultaneous release of target-specific exosomes with only one cleaving step. This specific example is described by figure 3 below. This example can generally be constructed in a way that only EVs/exosomes/viruses bound to two particles (i.e. the microparticles and the nanoparticle) are released for downstream characterization/quantification.

Advantageously, the presently disclosed isolation methods in general can be repeated such that cascades of isolations can be effected, using differently functionalised nanoparticles in order to obtain an ultra-refined selection of EVs (such as a highly specific sub-population of exosomes). Therefore, two, three, or even more surface biomarkers present on a target population of particles can be used in sequence in order to obtain a refined sub-population of target particles. Therefore, there is provided an advantageous approach for single discrete EV, exosome, or virus characterization from a large population of biomolecules.

The presently disclosed approach can also be used to initially select exosomes from a specific tissue and then, to identify disease-specific exosomes (i.e. exosomes expressing a biomarker indicative of disease) from those tissues. This is relevant because some disease biomarkers, identified in/on exosomes, are expressed in several tissues, yet only expression from some tissues is indicative of disease. For example, in Alzheimer's disease, amyloid Beta deposits in the brain may be used as a marker of the disease. Amyloid Beta also crosses the blood-brain-barrier and can be detected in blood. However, other organs such as the liver also produce amyloid Beta which is also released into the blood. By selecting, in a first instance, brain-specific exosomes such as those carrying NCAM, L1CAM or GLAST (which are not present in other organs), then a second selection step to identify those brain exosomes that carry amyloid beta we can guarantee that any amyloid β-containing exosome that is captured has originated from the brain, and not the liver. Furthermore, the presence of these brain-derived exosomes that express Aβ can be used to diagnose Alzheimer's disease.

Furthermore, the content of exosomes has been shown to change when organs or cells within an organism transition from healthy to diseased. Therefore, the same exosomes that originate from a particular organ can internally contain disease-markers. It is therefore beneficial to be able to isolate an intact exosome, and subsequently lyse the exosome in order to characterise the internal contents of the exosome.

Isolation Example 1: Photo-cleavage

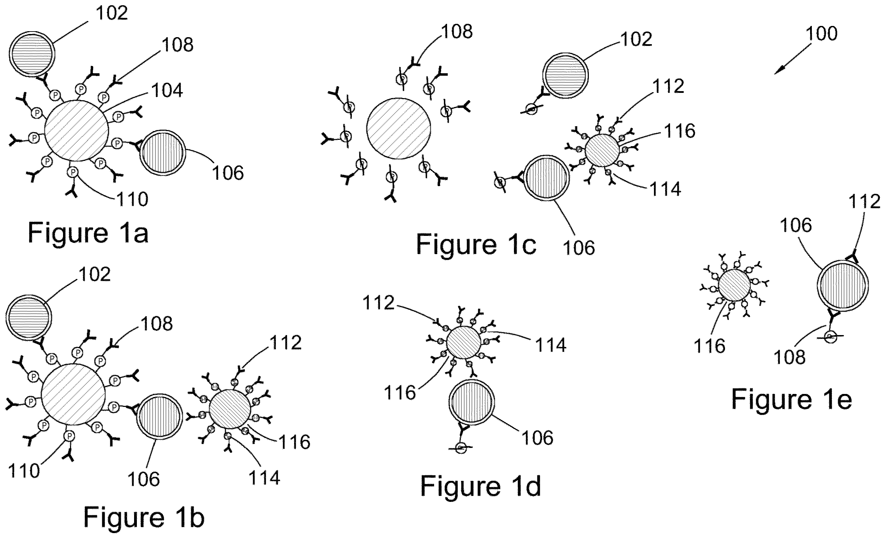

**Figure 1** show the steps of a first specific exosome isolation strategy 100, which uses magnetic microparticles 104 as exosome capture particles that are functionalised via photo-cleavable linkage 110 molecules. This example also illustrates the use of Gold nanoparticles (GNPs) 116 as target-exosome capture particles that are functionalised via disulfide linkages 114. The photo-cleavable linkage molecules 110 on the magnetic microparticles 104 are terminated with generic antibodies 108 (i.e. antibodies that are receptive to surface markers (e.g. transmembrane proteins) of exosomes or small extracellular vesicles (sEVs) in general). The disulfide linkages 114 on the GNPs 116 are terminated with a further antibody 112 that is receptive specifically to surface markers on the target exosome 106 (or small extracellular vesicle) of interest.

Beneficially, the disulfide linkages 114 on the GNPs 116 cannot be cleaved under the conditions used to cleave the photo-cleavage linkers 110 on the magnetic particles 104. Thus, two separate cleavage steps are possible.

Figure 1a illustrates an EV capture step, where the generic antibodies 108 on the microparticles 104 bind with all EVs, sEVs, and exosomes, including non-target EVs 102. In some examples, the generic antibodies 108 specifically bind only to sEVs and/or exosomes or even a specific population or subpopulation of exosomes.

Figure 1b illustrates a target-EV capture step, in which the GNPs 116 bind to the target-EVs 106 only. Thus, non-target EVs 102 or sEVs, which bound to antibody-terminated linkers of the microparticles 104, are not captured by the GNPs 116. Therefore, the result of this step is that the target EVs 106 are bound to exactly two particles: one microparticle 104 and one GNP 116.

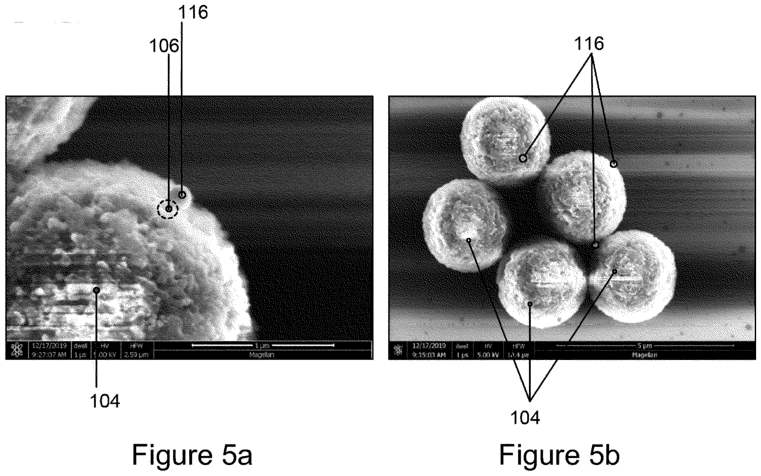

Advantageously, the ratio of the size (i.e., a diameter) of the microparticle 104 to the size of the EVs 102, 106 is designed such that bound target EVs 106 are dispersed evenly over the larger surface of the microparticle. Thus, no two EVs are proximate to each other, which has the advantage of ensuring that, in step 1b, the GNPs are very unlikely to bind to two target EVs. Preferably, a size dimension (i.e. diameter) of the microparticle 104 is about an order of magnitude greater than the EVs. This beneficial size ratio is described in more detail in Figure 5.

Figure 1c illustrates a first cleavage step wherein the photo cleavable linkers 110 are dissociated to release all EVs 102, 106 bound to the magnetic microparticle 104. The photo cleavage step may be effected by exposure to UV or near-UV light. The target EV is still bound to the GNP after the photo cleavage step, because the disulfide linkage molecules on the GNP are not photocleavable. The magnetic microparticles 104 are subsequently extracted by application of a magnetic field. The non-target EVs 102 and the target EVs 106 (each one bound to a GNP 116) remain in the mixture after extraction of the magnetic particles 104.

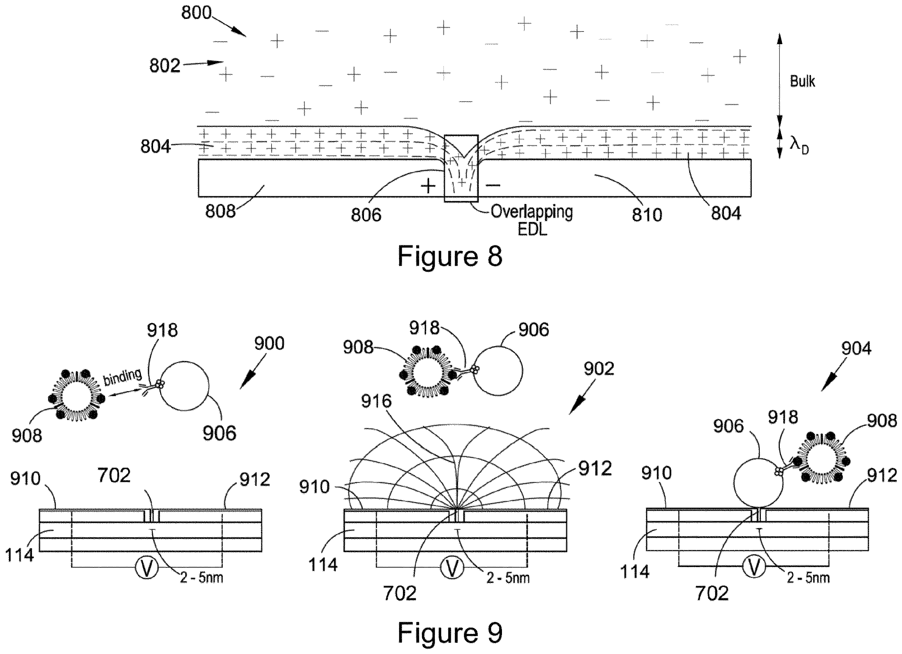

Figure 1d illustrates a separation step that separates the unbound non-target EVs from the target EVs 106 that are bound to the GNPs 116. Separation techniques may comprise centrifugation, which extracts the bound target-EV-GNP assemblies due to their greater mass and volume than the unbound EVs. Preferably, the separation is effected by application of an alternating electric field (not shown) which induces a dielectrophoretic force (DEP) acting on the GNP (and to a lesser extent to the EVs) 116. The GNPs 116 thus drag the bound target-EV 106 to a region from where the DEP originates (attraction) or to a region farther from it (repulsion). The DEP technique with the attraction feature is described in more detail below in Figure 9.

Figure 1e shows a second cleavage step in which the disulphide bond 114 linking the GNP 116 and the target EV 106 is cleaved. The cleavage is effected by treatment with a suitable reducing or alkaline reagent. For example, many suitable thiols can be used, where excess thiol is used in order to shift the equilibrium of the cleavage reaction to the right. Suitable thiols include as β-mercaptoethanol (β-ME) and dithiothreitol (DTT). Alkaline conditions (i.e., higher than pH 8) are also beneficial, in combination with thiols, to effect disulphide cleavage.

The second cleavage step of Figure 1 causes the target EV 106 to dissociate from the GNP 116. A further centrifugation or DEP step can then be used to separate the unbound GNPs from the unbound target EVs in order to extract the target. The extracted target EV 106 can then be used for further downstream applications such as lysing to characterise the internal and external contents of the EV. Alternatively, the steps of Figs. 1a to 1e can be repeated a plurality of isolated target EV 106, to obtain a refined sub-population of the target EVs. Specifically, a second GNP can be used with a further specific antibody that is receptive to a marker present only on sub-population of the extracted target EVs 106. In this way, a further refinement of the target EV population can be provided, in which a sub-population of the initial target EVs is extracted.

Isolation Example 2: Enzyme cleavage

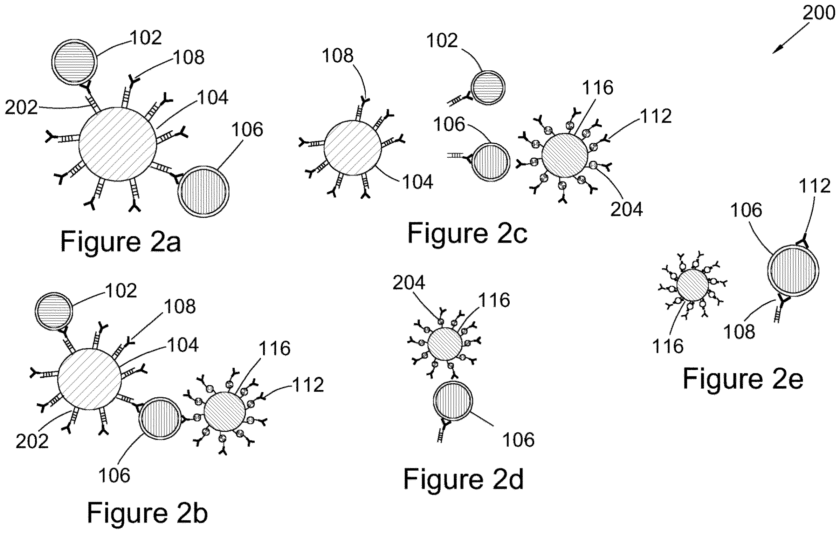

**Figure 2** shows the steps of a second specific exosome isolation strategy 200, which uses enzyme-cleavable linkages and disulfide linkages. Generally, the receptors 108, 112, the microparticles 104 and the GNPs 116 onto which the receptors are linked, are the same as described in Figure 1. Generic antibodies 108 (which alternatively are any suitable EV-specific binding agent protein) are attached to the microparticles 104 via RNA or DNA linkers 202. As a further alternative, the generic binding agents 108 may be specific to all exosomes, but not EVs generally, such that only exosomes (and not larger EVs) may become bound to the microparticles. Target-specific binding agents 112 (e.g. antibodies) are attached to the GNPs again via disulphide bonds. Beneficially, the disulfide linkages 113 on the GNPs 116 cannot be cleaved under the conditions used to cleave RNA or DNA linkers 202 on the magnetic beads 104. Thus, two separate cleavage steps are possible.

Figure 2a illustrates an EV capture step, where generic antibodies 108 on the microparticles 104 bind with all EVs, sEVs, and exosomes, including non-target EVs 102 and target EVs 106.

Figure 2b illustrates a target-EV capture step, in which the binding agents 112 of the GNPs 116 bind to the target-EVs 106 only. Non-target EVs 102 are not captured by the GNPs 116. Therefore, the result of this step is that the target EVs 106 are bound to exactly two particles: one microparticle 104 and one GNP 116. Similar to Figure 1, preferably the microparticles surface area is at least 100 times larger than the EVs 102, 106, such that it is very unlikely that any two target EVs 106 will be bound proximate to one another on the surface of the microparticle.

Figure 2c shows a first cleavage step wherein the enzyme-cleavable linkers are dissociated, resulting in the release all EVs 102, 106 bound to the magnetic microparticle 104. Cleavage is effected by treatment with an enzyme, for example a nuclease, suitable for digesting/cleaving RNA or DNA strands.

Figure 2d illustrates a separation step equivalent to that shown in Figure 1d. Non-target EVs 102 are separated from target EVs 106 that are bound to the GNPs 116. The separation uses any technique that exploits the size or density difference between the unbound non-target EVs and the (larger) target EV-GNP assemblies (e.g., centrifugation). Alternatively, the separation technique may exploit the conductivity of the GNP, e.g. using DEP to isolate the target EVs 106. In detail, the DEP is enabled by the relatively higher polarizability of the GNP relative to the surrounding medium.

Figure 2e shows a second cleavage step equivalent to that shown in Figure 1d, in which the disulphide bond 114 linking the GNP 116 and the target EV 106 is cleaved. Again, the cleavage is effected by treatment with a suitable reducing or alkaline reagent.

Isolation Example 3: Enzyme induced RNA/DNA hybrid cleavage

**Figure 3** shows the steps of a third specific exosome isolation strategy 300, which may be referred to a molecular beacon isolation strategy. Generally, the receptors 108, 112, the microparticles 104 and the GNPs 116 onto which the receptors are linked, are the same as described in Figure 1.

Generic binding agents 108 (e.g. antibodies specific to all exosomes) are attached to the microparticles 104 via a single strand of RNA or DNA 302a. In one embodiment, the length of this single strand nucleic acid would be between 50bp and 100bp. The single-stranded linker 302a is itself configured to bind with a second corresponding single-stranded RNA/DNA link (i.e. it's complimentary sequence). Target-specific binding agents 112 (e.g. antibodies) are attached to the GNPs again via RNA or DNA linkages 304. In one embodiment, the RNA or DNA linkages are preferably between 50 and 100bp. In addition, the GNPs 116 further comprise a tendril 306, having a sequence complimentary to the RNA or DNA linkage and forming a double strand duplex with that linkage, the tendril is further terminated with a single strand 302b of RNA or DNA. This single RNA/DNA strand 302b is configured to extend around a target EV 106 in order to reach its complimentary RNA/DNA strand 302a which forms the microparticle linkage. In one embodiment, the tendril 306 is between 200 to 1000bp, more preferably between 300 and 900 bp and even more preferably between 300 and 700bp. As such the length of the single strand RNA or DNA strand would be around 200-300nm in length and would be long enough to bind by proximity to the DNA/RNA probe on the nanoparticle but small enough so that the tendril does not bind to adjacent DNA or RNA molecules. Thus, the single RNA/DNA strand 302a on the microparticle is configured to bind with the single-stranded terminus 302b of the tendril 306 to form a double-strand of RNA/DNA 302c.

Beneficially, in this isolation strategy, only a single cleavage step is needed to isolate the target EV from the assembly formed of the magnetic microparticle, the target 106 and non-target 102 EVs, and GNPs. This is enabled by virtue of the fact that an enzyme is used to cleave RNA/DNA doubles-strand linkages, but which does not act on single-stranded RNA/DNA links. The method is as follows:

Figure 3a illustrates an EV capture step, where generic antibodies 108 on the microparticles 104 bind with all EVs, sEVs, and exosomes, including non-target EVs 102 and target EVs 106.

Figure 3b illustrates a target-EV capture step, in which the binding agents 112 of the GNPs 116 bind to the target-EVs 106 only. Additionally, the tendril 306, terminated with a single strand of DNA/RNA 302b, extends around the captured target EV 106 to reach the microparticle linkage strand 302a. The two single strands 302a, 302b, then bind to form a doubles-stranded linkage 302c.

Non-target EVs 102 are not captured by the GNPs 116. Therefore, the result of this step is that the target EVs 106 are bound to exactly two particles: one microparticle 104 and one GNP 116. Similar to Figures 1 and 2, preferably the microparticle are at around two orders of magnitude (at least 100 times) in terms of surface area larger than the EVs 102, 106.

Figure 3c shows the single cleavage step, which uses an enzyme 308 capable of cleaving only double-stranded RNA/DNA linkages. Thus, the RNA/DNA linkage 304 originally comprised of the GNP 116 and the newly-formed DNA/RNA linkage 302c are simultaneously cleaved. This has the advantage that the target-EV is released in a single step.

Figure 3d illustrates a separation step. In contrast to Figures 1d and 2d, the target EV 106 is already isolated (and unbound) at this point. Therefore, centrifugation alone is able to separate the target EV from the larger microparticle 104 and GNP 116. Alternatively, or additionally, a magnet may be used to extract the magnetic microparticles 104, and/or DEP may be used to extract the GNPs, in order to obtain a clean solution comprising only the target EV 106.

Furthermore, non-target EVs 102 are still bound (via a single strand of DNA/RNA 302a) to the microparticle, and are therefore extracted with the microparticle (by e.g. centrifugation or application of a magnetic field.)

Figure 3e illustrates the formation of a 'clean' solution comprising only the target EVs, after the Microparticles 104 and the GNPs 116 have been removed from the mixture. The EVs can then be assessed in downstream characterisation or quantified, as described below in more detail.

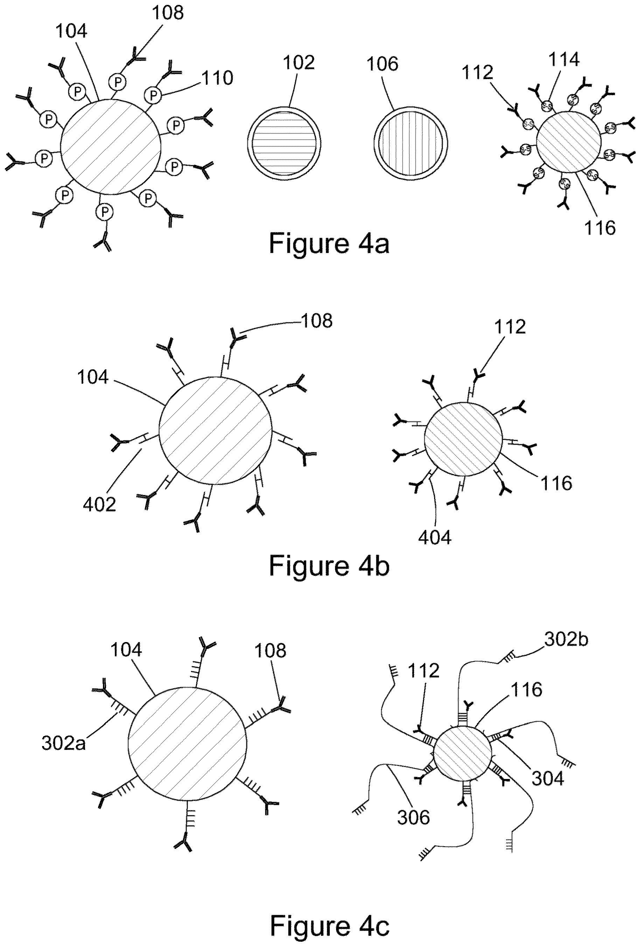

**Figure 4** show the individual nanoparticles, microparticles, and target biological entities - e.g. EVs or viruses present in the isolation strategies described above, and in relation to Figures 1 - 3.

Figure 4a shows the magnetic microparticles 104, otherwise referred to as an EV capture particle, a non-target EV 102, a target EV 106, and a GNP 116. The GNP is otherwise referred to as a target-EV capture particle. The microparticle 104 is functionalised with a ubiquitous binding agent 108, which is receptive generally to a subset of EVs, for example, all exosomes. Thus, larger EVs, proteins, and cell fragments, will not become bound to the microparticles via the generic EV binding agent 108. Preferably, the generic binding agent 108 is an antibody, which is receptive to a surface (transmembrane) protein on the surface of a subset of EVs. Other generic binding agents may include antigen-binding fragments or aptamers.

Where the EVs of interest are exosomes in general, the generic binding agent 108 may be receptive to tetraspanins on the surface of the exosomes. Alternatively, the microparticles may be functionalised in order to bind with viruses (i.e. virions) in general.

The general binding agent 108 is attached via a photo-cleavable linker 110 in Figure 4a, in accordance with the isolation strategy described in Figure 1. This photo-cleavable linker can generally be dissociated by exposure to US or near-UV light.

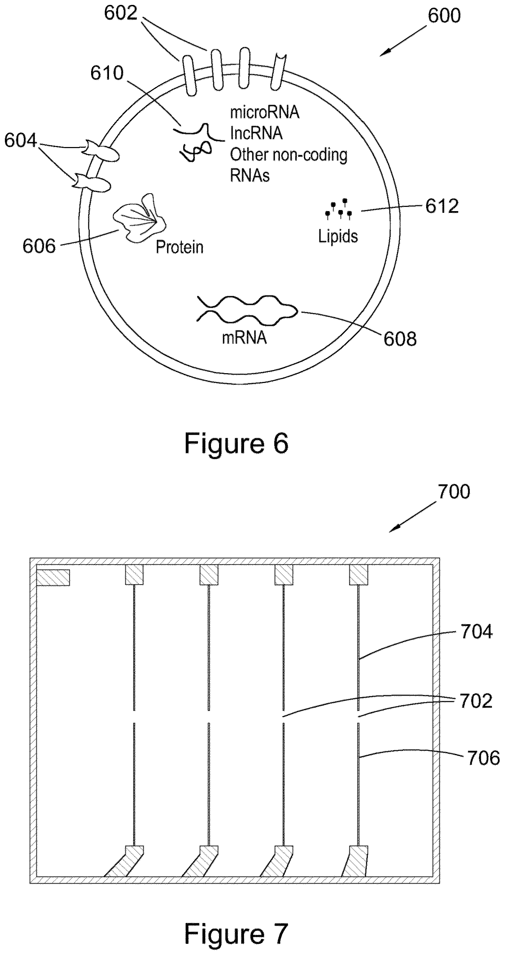

The GNP is functionalised with a specific binding agent 112, which is receptive to a (target) subset EVs 106 within the larger set of generic EVs 102 captured by the microparticle 104. Again, preferably the specific binding agent 112 on the GNP 116 is an antibody that is receptive to an antigen/protein on the surface of the target EV 116. For example, the target EV may be an exosome that is indicative of a particular disease or cancer, where the specific binding agent 112 is receptive to a disease biomarker. For example, the HER2 protein can be targeted as a disease biomarker, which is present on exosomes associated with breast cancer. For example, the HER2 protein may be marker 604 as shown in Figure 6. In this example, the specific binding agent 112 on the GNP 116 is a HER2-bidning agent, such as Herceptin (Trastuzumab) or a HER2-binding fragment thereof. The specific binding agents 112 are attached to the GNPs 116 via disulfide bonds 114 that cannot be cleaved by UV light. The disulphide linkers 114 are cleaved in alkaline conditions with a suitable reducing agent, such as a thiol.

Figure 4b shows a further microparticle 104 and GNP 116, respectively functionalised with generic 108 and specific 112 binding agents. This figure illustrates that generally the binding agents can be functionalised with any suitable linker 402, 404. Preferably, the linkers 402, 404 on the microparticle and the GNP, respectively, are different such that two separate cleavage steps can be effected. This is advantageous because it allows the microparticle to be cleaved from the target EVs 106 and extracted (e.g. by application of a magnetic field), before the GNPs 116 are cleaved in a second step from the target particles 106. After the second cleaving step, the GNPs 116 are extracted via centrifugation, or DEP, in order to isolate the target EVs, or target viruses 106.

Figure 4c shows the microparticle 104 and GNP 116 consistent with the method used in Figure 3, i.e. the molecular beacon method. The single strand of RNA/DNA 302b which terminates the tendrils 306 of the GNP 116, are configured to bind with the single strand RNA/DNA linkages 302a on the microparticle. Thus, the linkages 302a of the microparticle cannot be cleaved by an RNA/DNA cleaving enzyme 308 until a double strand (not shown in Figure 4) of DNA/RNA is formed.

**Figure 5** shows a scanning electron microscope image of magnetic beads 104 with highlights showing positions of bound target vesicles 106, captured by GNPs 116. In general, the magnetic microbeads 104 function as EV capture particles, and the GNPs function as target-EV capture particles 116, which capture only a subset of the particles captured by the microparticle 104.

In general, the size ranges of the particles is as follows:

- Target EVs 106 and EVs in general 102: 40 - 160 nm
- Magnetic microparticles: 2 - 3 $\mu$m in diameter, when spherical (which is not necessarily the case)
- GNP / target-EV capture particles: 50 to 300 nm in diameter, when spherical.

The relative size of the microparticle 104 to the GNP 116 can be seen in Figure 5. As mentioned above, the surface area ratio of microparticle 104: GNP 116 is at least 100, but can be up to around a 1000 times greater. Consistent with the above size ranges, in one example, the GNP is 200 nm in diameter and 0.13 $\mu$m$^2$ in surface area, and the microparticle is 2.7 $\mu$m in diameter and 22.9 $\mu$m$^2$ in surface area, such that the surface area ratio between the GNP and the microparticle is 182.

It not necessary for the particles to be spherical as shown. However, it is advantageous for the size dimension of the microparticle is great enough such that the microparticle104 has a substantially larger surface area that that of the GNP 116. A large surface area on the microparticle creates a large amount of available binding space for EVs. Thus, even at high concentration of EVs, it is unlikely that two EVs will bind proximate to each other on the surface of the microparticle. In more detail, no two EVs will bind on the microparticle surface at a separation of less than 300 nm, or, the size dimension of the GNP. Thus, it will be appreciated that it is very unlikely that two EVs (generic 102 or target 106) will bind onto the surface of the microparticle 104 such that a GNP is able to bind to both EVs.

It is therefore beneficial to keep the size of the GNP relatively small (at least 10 times smaller in length) compared to the microparticle, to reduce the likelihood that a GNP is able to bind to two EVs at once. By maintaining the above ratio between microparticle and GNP size, it becomes vanishingly unlikely that a GNP will bind to two EVs.

After an isolation method has isolated a target EV in accordance with either Fig. 1d or Fig 2d, it will therefore be appreciated that each target EV 106 is bound to exactly one GNP 116. This is particularly beneficial for quantification sensing purposes, as it is possible to detect, precisely, the number of EVs captured by measuring a current. The GNPs, bound to the EVs, enable an electrical current to be detected. This quantification sensing is derived in detail below, corresponding to Figures 7-9.

**Figure 6** illustrates the general structure of an exosome 600, including general markers 602 and more specialised/specific (e.g., disease or tissue-specific) markers 604. Generally speaking, exosomes provide indicators for a variety of biological responses in humans and other organisms. Exosomes are associated with various diseases including cancer, and diseases involving the cardiovascular and nervous systems. Exosomes are understood to provide a variety of functions within humans, for example: removal of cell constituents, and potentially as a mechanism to communicate between cells as part of a regulatory system.

Exosomes comprise a cell membrane made of a lipid bilayer, as shown, and are therefore a subset of extracellular vesicles (EVs). Exosomes are generated by cells and therefore contain, within the boundary of the lipid bilayer, a variety of cell constituents including: nucleic acids, proteins 606, lipids 612, RNA and DNA fragments, and various subcategories of RNA and DNA 610 (e.g., mRNA 608, microRNA, Y-RNA, mtRNA, mtDNA, dsDNA, ssDNA, and the like).

Furthermore, the surface of exosomes carries various hallmarks 602 that are exosome-specific, and therefore allow exosomes to be differentiated from other EVs, and cell fragments. These hallmarks 602 include tetraspanins, other GPI-anchored (Glycosylphosphatidylinositol anchored) proteins, integrins, and proteins with lipid or membrane protein-binding ability. Furthermore, other cytosolic or periplasmic proteins with lipid/membrane-binding ability may also be targeted, for example, ESCRT-I/II/II (such as TSG101 or CHMP), or accessory proteins such as ALIX.

Tetraspanins found on the surface of exosomes in general includes: CD9, CD63, CD81, CD326, CD82, CD37 and CD41. Tetraspanins demonstrate the presence of a lipid bilayer. Other surface-anchored proteins that are typically universal to exosomes includes the following, which is not an exhaustive list (MHC class I (HLA-A/B/C, H2-K/D/Q), integrins (ITGA/ITGB), transferrin receptor (TFR2), and Heparan sulfate proteoglycans.

The ability to isolate exosomes based on these hallmarks 602 avoids the accidental isolation of contaminants such as lipoproteins, protein aggregates and exomers. In other words, the ability to reliably capture and isolate exosomes on the basis of exosome-specific surface proteins significantly reduces the probability of a false positive.

Referring back to Figures 1a, 2a, and 3a, a general population of EVs including target and non-target EVs 102, 106 is typically sequestered based on binding with any one of the above mentioned universal/hallmark surface markers 602. Subsequently, corresponding to figures 1b, 2b, and 3b, the target-capture particles (i.e., the GNP 116) bind to a specific membrane biomarker that is present only on a subset of the initially captured EV. Examples of specific biomarkers 604 include proteins expressed by certain diseased cells, including GPC1 for various cancers, and HER2 for specifically breast cancer.

It will therefore be understood that a subpopulation of EVs or exosomes can be isolated based on specific biomarkers 604, which may be disease-specific biomarkers. Therefore, beneficially, the mere detection of the presence of this subpopulation of EVs or exosomes 600 can immediately indicate a disease.

Another advantage of the present method is that the isolation and detection maintains the integrity of the exosome, and its contents (606, 608, 610, 612) are not lost. Therefore, an isolated population of exosomes can be lysed to characterise the complete content of the exosome, including the internal contents (i.e., proteins 606, mRNA 608, noncoding RNAs 610, and lipids 612). Thus, in addition to the isolation process which itself can indicate certain diseases, further characterisation of the internal contents can be used to provide confirmation of said particular disease, or provide a first indication of a disease/condition. In this regard, the following disease-specific biomarkers are known to be cytosolic (i.e. internal) and may be used to indicate or confirm a disease: Microtubule-associated Tau; Heat shock protein HSP70, and various other DNA, RNA, lipids and proteins.

Apparatus for extracting and sensing GNP particles bound to EVs or Viruses

**Figure 7** shows an array of five electrode pairs with a nano-gap, i.e. a separation between the tips of the electrodes of around 100 nm, or preferably less.

Each of the nano-gaps 702 defines a sensing region, or a DEP focusing region, dependent on the mode of operation of the device. Each of the electrodes 704, 706 of each electrode pair are typically gold, and are fabricated such that the tips of the electrodes approach each other in order to produce a nano-gap of less than around 100 nm. An array of sensors 700 as shown here may comprise far more than 5 electrode pairs. For example, more than 10 or 100 sensors 700 may be used, each comprising a plurality of sensing/DEP regions 702.

In use, for example to attract and characterise the presence of GNP-exosome assemblies, an alternating current can be applied to the sensor 700, which in turn induces an alternating electric field emanating from the sensing regions 702. The electric field may be used to attract GNPs and concentrate them around the sensing region 702.

Each sensing region 702 may then be used to apply a direct current to characterise a response of each of the regions, to identify and quantify the presence of GNPs.

**Figure 8** illustrates a sectional view of an electrode pair with a nano-gap in a model liquid containing charged entities. An electrical double layer is present in fluid immediately adjacent to the electrodes 808, 810. In this sensor example 800, individual ions 802 persist in the medium, and concentrate over the electrodes' 808, 810 surface to form the double-layer 804. This double layer can be seen to overlap in the sensing region between the electrodes 806.

Advantageously, the separation between the electrodes 808, 810 is small enough such that the electrical double layer (EDL) present over each electrode 808, 810 overlaps in the sensing region. That is, the EDL overlaps in the nanogap 806. This overlap is beneficial for capturing of targets (i.e. gold or other conductive nanoparticles), and the subsequent sensing/quantification of said target particle. In other words, it may be favourable for the lateral distance between the pair of electrodes may be less than twice the width of the EDL in the surrounding solution. In some implementations, the lateral distance between the pair of electrodes may need to be sufficiently small in order to ensure this.

Generally, an EDL is a structure that appears on the surface of a charged surface object when it is exposed to a solution with dissolved ions. The double layer refers to two parallel layers of charge surrounding the surface (e.g., the electrode surface). The first layer comprises ions adsorbed onto the surface due to either chemical interactions and/or a charged electrode surface. The second layer is composed of ions attracted to the first layer via the Coulomb force, where the ions electrically screen the first layer. In this way, the width of the EDL may be seen as equivalent to the Debye length in an ionic solution. The Debye length is generally a property of an ionic solution, and is a measure of a charge carrier's net electrostatic effect, and to what extent said effect persists in the solution. Every Debye-length $\lambda_D[m]$, the electric potential will decrease (i.e. be screened) in magnitude by 1/e. The width of electrical double / Debye layer is a function of at least the ionic strength of the fluid. Moreover, the Debye length may be on the order of a few nanometres on an electrode having an applied potential. In implementations, a gap 102 between the electrode pair may need to be less than ~ 20 nm in order for the Debye length (i.e. EDL width) to be equal or less than half the lateral distance between the pair of electrodes. In some implementations, even narrower gaps 102 may be created, e.g. as low as 5 nm or even 2 nm gaps.

The Debye length is related to ionic strength of the fluid, according to:

$$\lambda_D[m] = \sqrt{\frac{\varepsilon_0 \varepsilon_T k_B T}{\sum (z_i q)^2 c_i}}$$

where z represents ionic species, and q their corresponding charge values.

Alternating electric fields may be used to induce an electrophoretic force on conductive nanoparticles bound to targets EVs/particles. Thus, conductive nanoparticles such as GNPs may be actively transported to, and concentrated around, a sensing region 702, 806 or nanogap of an electrode pair.

In detail, the time averaged DEP force of a spherical particle with radius R and in a solution with a dielectric permittivity of $\varepsilon_m$ is provided below:

$$F_{DEP}(\omega) = \pi \varepsilon_m R^3 \mathrm{Re}\big(f_{CM}(\omega)\big)\nabla |E|^2$$

The 'real' part of the above Clausius - Mossotti factor (CMF), $\mathrm{Re}(f_{CM}(\omega))$, determines the direction of the DEP force based on the dielectric permittivity and conductivity (inside the CMF term) of the solution and particle. With conductive nanoparticles, this force is generally attractive between the nanoparticles and the source of the electric field. The gradient of the E-field in solution squared, $\nabla |E|^2$, correlates with the supply voltage applied across the DEP electrodes. The DEP electrodes may also be the same electrodes 910, 912 as used to characterise and measure the sensing region to detect the presence of the target entity. Additionally, the magnitude and the sign (i.e. attractive or repulsive) of the DEP is also a function of permittivity of a particle (not only conductivity), especially for biological targets. Furthermore, the DEP force can be a function of the frequency of the applied E-field, especially for biological targets. However, for metallic particles, DEP force is generally invariant with respect to the frequency of the applied E-field.

Advantageously, dielectrophoresis (DEP) offers rapid concentration and isolation of nanoparticulate matter that does not depend on specific chemical binding or alterations. The DEP process commonly utilizes two electrodes in solution that are subjected to an alternating electric field (E-field). The force on the particles derives from the fact that the alternating electric field induces local dipoles within the particles. These local dipoles cause a net force toward, or away from, the E-field gradient depending on: the frequency of oscillation, and the relative dielectric permittivity of the particle and surrounding medium.

Detecting Extracellular Vesicles (EVs), by known techniques can require purification by ultracentrifugation or precipitation, and detection through Nanoparticle Tracking Analysis (NTA) or Dynamic Light Scattering (DLS). Both these measure the Brownian motion of nanoparticles, whose speed of motion, or diffusion constant, is related to particle size through the Stokes-Einstein equation. Therefore, such devices are not sensitive to small concentrations of extracellular vesicles such as exosomes and provide almost no information relating to proteins or other biomarkers decorating the surface of EVs.

There is now described a more sensitive system and method, which can produce a high signal-to-noise ratio not only in detecting e.g. exosomes but also in transporting relevant biomarkers.

**Figure 9** illustrates an example of a method of using dielectrophoresis (DEP) to influence and accelerate the transport of nanoparticle-entity assemblies/pairs. Figure 9 further illustrates the capture and characterization of the target entity 908 with the aid of gold nanoparticles 906. The electrodes 910, 912 are gold in the illustrated example, with a nano-gap of approximately 40 - 50 nm, although this may be as low as 10 nm or even 5 nm. The electrodes are provided on a substrate 114.

Target entities 908, such as exosomes, forming part of a biofluid may be sequestered by functionalised nanoparticles 906 as described above. In the example of figure 9, the nanoparticles 906 are gold nanoparticles (GNPs). In accordance with the isolation methods described above in relation to Figures 1 - 3, the assemblies comprised an EV 908 and a GNP 906 will preferably comprise no more than two particles.

Step 900 shows a binding/sequestering event taking place in order to form a nanoparticle-target assembly. The microparticle 104 and the steps used to extract and isolate the target particle 106, 908 are not shown illustrated for simplicity.

Step 902 depicts an electric field 916 being applied to the medium such that a force is exerted on the nanoparticle(s) 906. For a DEP force to be exerted on the nanoparticles 906, a relative difference should exist between the conductivity of the nanoparticles and the surrounding medium as described above. Thus, in implementations, GNPs are beneficial because they are more conductive than the dilute medium in which they are suspended. The DEP force draws the GNP 906 and the target 908 towards the sensing region 702. An alternating current should be applied to induce an alternating electric field. In some implementations a frequency of up to about 1.5 MHz may be used.

Step 904 shows the electrophoretic/DEP causing the nanoparticles 908 to be concentrated around the sensing region. Advantageously, the electrodes 910, 912 do not need to be functionalised with antibodies or any binding agent, because GNPs have a natural affinity, i.e. a thermodynamically favourable interaction, with gold electrodes. Therefore, when the nanoparticles 908 become concentrated around the medium due to DEP, the GNPs become effective bound to the region 702 in between the electrodes.

An increasingly narrow lateral separation of electrodes may increase the dielectrophoretic (DEP) force without the need to increase a voltage to power the applied electric field. However, with DEP there can be adverse effects on the sample solution and its analytes due to large trapping voltages. In order to overcome the thermal motion of particles with dimensions under 100 nm, conventional DEP electrodes with micrometre-scale gaps typically require an unfavourable high trapping voltage, for example, a minimum of 10 V. Such large trapping voltages can cause Joule heating, bubble formation, and unfavourable electrochemical reactions.

Implementations of the described system/method have small gaps between the electrodes, e.g. less than ~ 10 nm, to facilitate bridging this gap with one or a few nanoparticles. This can provide an additional advantage of increasing the DEP trapping force without the need to raise the trapping voltage, mitigating these unfavourable effects. Further, by reducing the width between DEP electrodes, the gradient of the E-field can be increased substantially, which provides a greater force on the GNPs and thus an improved ability to efficiently concentrate the GNPs/nanoparticle-entity assemblies around the sensing region. Ultimately, this can result in a method more sensitive to the presence of target entities, since the nanoparticle-entity assemblies may be collected more efficiently.

An AC current can then be applied to the electrodes 910, 912 that induces an alternating E-field in the fluid suspension containing the nanoparticle-entity assemblies in step 902. After the attraction 902 and concentrating 904 of the nanoparticle-entity assemblies around the sensing region 702, the assemblies bridge the electrode nanogap 102. A direct current is generally then applied, which is used to characterise/measure a response of the sensing region in order to identify whether the assemblies (and thus exosomes) are present. A voltage of around 1 V may be applied to produce a direct current. A baseline current (where no bridging occurs) may be around 1 - 20 pA. A current produce from a bridged gap (i.e. as seen in step 904) may be around 1 -100 nA. Thus, a signal to noise ratio of over a thousand may be achieved in implementations of the described method. Furthermore, when bridging occurs, an activation voltage can be applied which fuses the bridging particles, thus decreasing their resistance; in such examples, currents of 1 mA and above can be carried by the fused bridging nanoparticles.

An Ohmic response (i.e. indicative of classical resistor behaviour) generally indicates that the nanogap sensing region 702 is bridged by GNPs. Therefore, a characteristic linear relationship may be seen when a direct current is applied across the electrodes. However, in other implementations, an AC current may be used to characterise a response of the sensing region. In implementations, the same AC current as used to induce a DEP force in the nanoparticles may be used to characterise an impedance response. In this way, an Ohmic response may still be identified for example, by identifying a characteristic impedance profile of a classical resistor. As a further example, when very narrow electrode gaps are fabricated on the order of 5 nm, non-classical electron transport effects may be seen upon application of a constant voltage to an electrode pair bridged with nanoparticles. Thus, if effects such as tunnelling and flickering resonance are to be expected, the identification of nanoparticle-entity assemblies may comprise identifying a non-classical current-response profile for a resistor.

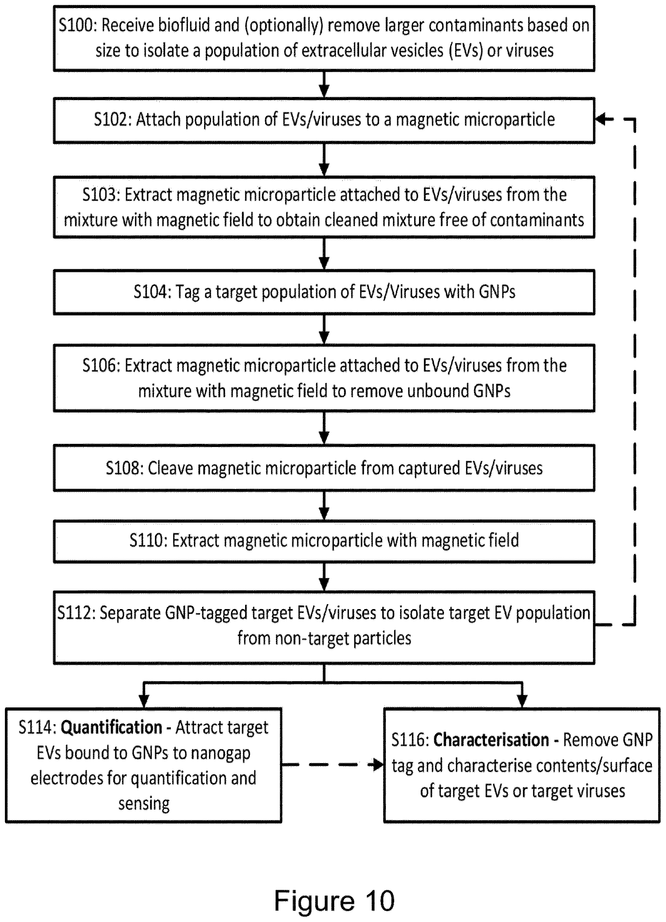

**Figure 10** shows a flowchart which describes the process of isolating a target population of biological vesicles from a biofluid. It will be understood that the target population can be any of a particular family of EVs, a population of exosomes deriving from a particular organ (e.g. liver) or indicative of diseased cells, or a specific virus. The biofluid may initially EVs of varying sizes, a plurality of different exosome populations, viruses, and the like. The biofluid may further comprise a number of contaminants including: open cell fragments, lipoproteins such as low-density and high-density lipoproteins (LDLs, HDLs), cholesterol, protein aggregates, Ectosomes. This list is not exhaustive.

Step S100 can optionally comprise an initial filtration or isolation step in which large biological particles and contaminates are removed. Thus, suitable separation techniques exploit the relative size of particles, and include: centrifugation, and linear-flow within a microfluidic device that exploits different hydrodynamic radius of particles. Large EVs, large proteins or aggregations can thus be separated from a population of EVs or viruses within which the target population is contained.

Step S102 comprises sequestering or capturing a population of EVs, e.g., capturing exosomes or small EVs of a certain size, using micro-sized capture particles that may be magnetic microparticles. Such microparticles 104 are described in detail in relation to figures 1 - 3.

In Step S103, a cleaning step is performed (using a magnetic field), where the magnetic microparticles are extracted from the mixture in order to separate them from contaminants (including unbound cell fragments, unbound antibodies, and unbound targets). Thus, a cleaned mixture may be obtained prior to S104, where GNPs are introduced.

In step S104, functionalised GNPs are introduced to the mixture, where the GNPs bind to a specific subset of EVs. This step corresponds to Figs. 1b, 2b, and 3b described above.

In step S106, the microparticles are again extracted from the mixture, carrying with them the captured EVs/viruses and target EVs (or target viruses), in order to remove further unwanted contaminants, which in this case include unbound GNPs. This extraction is preferably carried out by application of a magnetic field. However, due to the relative size of the assemblies formed by the microparticles bound to EVs/viruses, centrifugation may also be used, which discriminates based on particle size.

As mentioned above, two 'cleaning' steps are performed, both involving application of a magnetic field to sequester the magnetic microparticles and the particles to which they are bound. Sequestering the microparticles thus allows other contaminants in the mixture to be cleaned/washed. For example, in S106, unbound GNPs (the GNPs having been introduced in S104) may be washed away during the extraction of the magnetic microparticles.

The flowchart in Figure 10 shows two cleaning steps (S103 and S106, which remove contaminants and unbound GNPs, reactively), which is the preferable example of carrying out the isolation method. Alternatively, the GNPs may be added to the mixture to tag the target particles prior to Step S103. In this alternate example, only one cleaning step would be required (i.e., in S103) which would simultaneously remove contaminants and unbound GNPs, and may obviate the need to carry out S106. Irrespective of this, however, the result of the cleaning steps is that a cleaned solution is formed by step S103 which preferably is free from contaminants such as open-cell fragments and larger EVs, or non-target viruses, as detailed above.

In step S108, the mixture is treated to cleave the magnetic beads away from the particles (EVs and/or viruses) which were sequestered in step S102. Preferably, cleavage is effected by application of UV or near-UV light, which dissociates photo-cleavable linker molecules used to functionalise the microparticles.

In step S110, the microparticles are extracted or separated from the mixture by application of a magnetic field. Following this step, there remains a mixture comprising the target particles tagged with GNPs, and un-tagged non-target particles. This corresponds to fig. 1d and fig. 2d

In step S112, the GNP-tagged target particles are separated from the non-target particles. This may be effected by application of an alternating electric field to induce a dielectrophoretic force (DEP) on the tagged-particles only. DEP is described in detail above in relation to Figs. 7-9.

After step S112, two alternative applications can follow:

In step S114, the assembly of the tagged target particles is maintained such that the presence of the GNPs can be exploited. Specifically, the GNPs are drawn towards an array of electrodes, for examples as shown in Figure 7, where an electrical response to an applied current is sensitive to the number of GNPs in the sensing regions 702 of the electrodes. This method of sensing and characterisation using electrodes and DEP is described in detail in related published PCT application, publication number WO 2019/211622 A1.

The target particles are bound to the GNPs in a 1:1 ratio (by virtue of the relative sizes of the microparticles to the GNPs, as described above in relation to Figure 5) therefore, the number of target particles can be quantified accurately. Beneficially, the target particles can be used subsequently in S116 after S114 because the integrity of the target particles is maintained.

Step 116 involves the characterisation of the target particles. The GNP is cleaved from the target, and extracted via either DEP or centrifugation, for example. The method of cleavage depends on the nature of the linkage used on the GNP; for example, a disulfide linker can be cleaved as described above using suitable reducing agent in alkaline conditions. The surface proteins of the target particles may be characterised without destroying the vesicles, for example, by ELISA (enzyme-linked immunosorbent assay) or other suitable characterisation technique. Furthermore, or alternatively, the target particles can be lysed (e.g., using a simple detergent) in order to free the contents of the vesicles, e.g., cytosolic proteins and RNA etc. The contents can then be characterised using suitable techniques including: ELISA, PCR and RNA/DNA sequencing, mass spectrometry (i.e. for protein characterisation. No doubt many other techniques for characterisation of biomolecules in general will be apparent to the skilled person.

Advantageously, because the steps of the method S102 to S112 maintain the integrity of the target particles, the steps may be repeated as indicated in order to produce a further-refined population of particles. Merely for examples, a first pass of steps S102 to S112 may isolate exosomes, from a biofluid mixture containing many non-target exosome families and EVs, which are derived from brain tissue. The brain tissue exosomes isolated by S112 may then be re-introduced in step S102 (after removing the tagging GNP). The steps S102 to S112 are then repeated to isolate a subpopulation of brain-tissue exosomes that contain amyloid-beta deposits.

This is particularly beneficial because other organs, e.g., the liver also, produce amyloid Beta. Thus, if amyloid beta-containing exosomes were targeted in the first instance, a non-specific population of exosomes may be captured which derive from multiple different organs (i.e. liver and brain). By selecting in a first instance brain-specific exosomes (which are not present in other organs), and subsequently targeting amyloid beta-containing exosomes, an improved level of specificity is achieved in which a refined sub-population of exosomes can be obtained. Amyloid Beta can cross the blood-brain barrier and can be indicative of Alzheimer's disease. Recycling isolated target particles from S112 to S102 is therefore beneficial in diagnosing diseases or conditions that are otherwise difficult to diagnose.

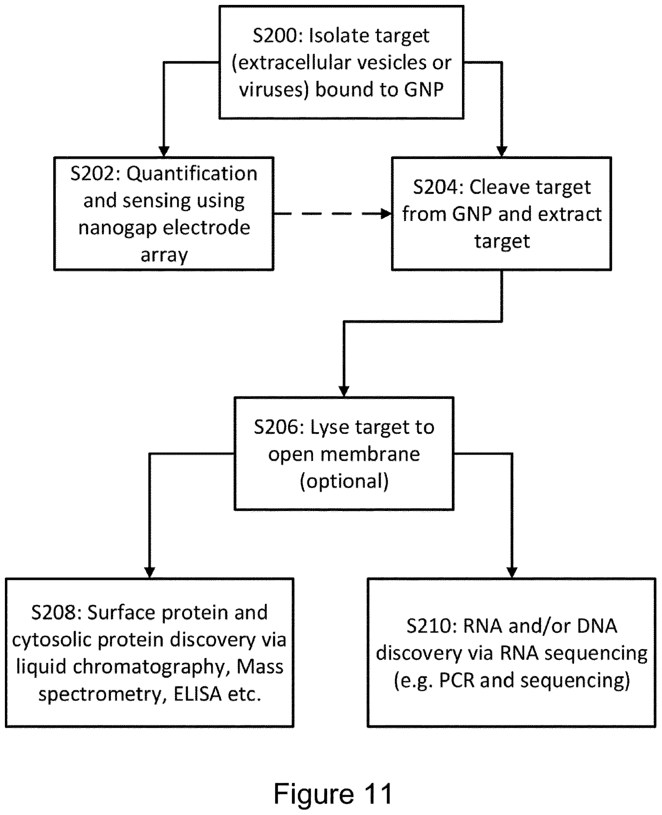

**Figure 11** shows a more detailed workflow for steps S114 and S116. In step S200, isolated targets are obtained still bound to the GNPs. As mentioned above, target particles can either be quantified in S202 using the electrode array described in Figures 7 - 9, or the targets can be cleaved from the GNPs in step S204. S202 can optionally be followed by cleavage from the GNP and the rest of the steps followed. In step S206, the target particles are lysed (i.e., the cell membranes are destroyed) such that the internal contents can be characterised together with the surface proteins/markers. Nevertheless, it should be appreciated that S206 is optional, and that lysing is not necessary in order to perform the characterisation steps in S208.

Steps S208 and S210 can both be performed, and are not mutually exclusive to one another. Larger proteins are characterised in S208 using techniques such as mass spectrometry or gas chromatography. In step S210, RNA and DNA comprised within the target particles can be identified using sequencing techniques, optionally with suitable amplification techniques such as PCR. Thus, steps S208 and S210 can be used for novel or previously unknown biomarkers or proteins within target populations of EVs. The discovery of novel biomarkers on, for example, exosomes can allow a new sub-population of exosomes to be targeted (i.e., with GNPs functionalised with suitably specific antibodies) based on the novel markers.

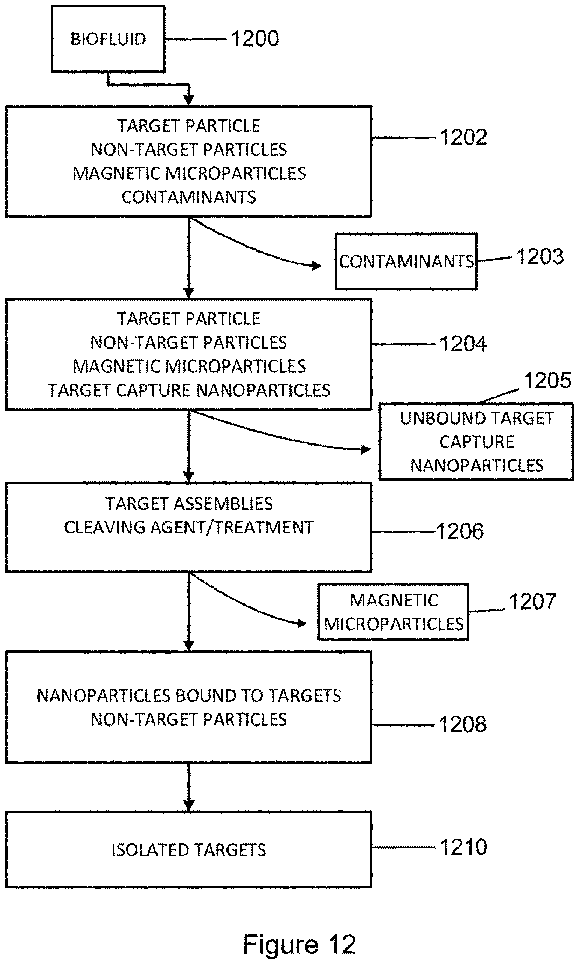

**Figure 12** shows a model system to carry out the steps of the any of the examples isolation methods described above. Each successive step shown can be performed in a new vessel. However, this is not necessary, and alternatively the whole method may be performed in a single vessel, where any contaminants etc. are extracted and discarded during the method. Furthermore, the method may be carried out in a single apparatus formed from a microfluidic device, in which successive compartments or chambers of the microfluidic device provide a space/volume for each step of the method. It will be understood that the target particle may be a particular type of EV, an exosome, a virus or retrovirus (the list is not exhaustive).

A biofluid 1200 is obtained containing the target entity. The Biofluid 1200 may be obtained from a patient or animal, or may have been obtained from a cell culture. The biofluid 1200 is introduced to a first chamber 1202 along with magnetic microparticles, which in this example act as universal capture microparticles. For example, the magnetic microparticles may be functionalised to capture all EVs, or all exosomes, or a specific class of viruses or retroviruses. First container 1202 defines a precursor mixture, where contaminants are still present.

Assemblies as seen in Figs. 1a, 2a, and 3a form in this precursor mixture 1202. Once the assemblies are formed, a magnetic field is applied to the precursor 1202 in order to extract the assemblies from the rest of the mixture, including the contaminants of the biofluid 1200. The contaminants 1203 are thus separated from the mixture and are discarded.

A cleaned mixture 1204 is formed after magnetic extraction of the microparticles assemblies, and the contaminants 1203 have thus been separated from the biofluid. A plurality of target capture nanoparticles, which may be gold nanoparticles (GNPs), are added to the clean solution. The nanoparticles are functionalised such that they bind only to the target particles on the microparticles assemblies. After addition of the target capture nanoparticles, assemblies as shown in Figs. 1b, 2b, and 3b are formed in the cleaned mixture 1204.

Prior to cleaving the linkers on the assemblies, mixture 1204 is treated again (in accordance with step S106) to remove any unbound target capture nanoparticles 1205, which are discarded.

The cleaned mixture 1204 is then treated in order to cleave the linkers attaching the target and non-target particles to the magnetic microparticles. Thus, a mixture with a new composition is formed in 1206 after the cleaving. It will be understood that 1204 and 1206 may be the same vessel/container, where the cleaving comprises merely exposing the cleaned solution 1204 to UV or near-UV light (in accordance with Figure 1), in which case no agent is added. Consistent with Figure 2, a nuclease cleaving agent may be added in mixture 1206. The constituents of mixture 1206 are illustrated according to the examples in Figs. 1c and 2c.

A second magnetic field is applied in order to extract the now-unbound magnetic microparticles 1207 from the mixture 1206. The result of extracting the microparticles is a mixture containing unbound non-target particles, and target particles bound to the target capture nanoparticles. Either dielectrophoresis or centrifugation is applied to mixture 1208 in order to extract the nanoparticles and thus isolate the targets 1210. For example, the bound nanoparticle-target assemblies may be attracted to an electrode array consistent with Figure 7, allowing the unbound non-target particles to be washed away. The result is thus a mixture containing only the target particles and the nanoparticles. In accordance with steps S200 onwards of Figure 11, the nanoparticles can be cleaved away from the targets, for further downstream applications, e.g., quantification, sensing, characterisation etc. of the targets and their biological contents.

Additional examples

A number of examples are provided above for means of cleaving a linkage (e.g., disulfide link 114) between target capture nanoparticles 116 and target EVs 106. For example, disulphide cleavage may be effected by treatment with a suitable reducing or alkaline reagent. In other examples, acid-cleavable linkages may be used to link nanoparticles 116 with binding agents 112. Generally, therefore, pH sensitive linkages (e.g. acid-sensitive or alkaline-sensitive) may be used to connect capture nanoparticles 116 and target EVs 106, where either acidic or alkaline reagents may be used to cleave said link. Such methods are referred to as pH elution methods.

In further general examples which use pH elution, no specific linkage molecule (in its place, e.g. a link that it suitable for pH-sensitive cleaving) is required to connect the capture nanoparticle 116 to a binding agents 112. In such examples, a cleaving agent acts directly on the binding agent 112. For example, the binding agent 112 itself may be denatured or detached from the target EV by treatment with a suitable acidic or oxidising reagent.

In a specific embodiment, in one method of isolating target EVs, one important difference to the previous series of results is that the antibody used for EVs capture on the magnetic beads (MBs) is first modified with an amine-reactive NHS ester, coupled to PEG4, used for spacing, and to dibenzocyclooctyne-amine (DBCO), used for the click reaction with azide (N3)-modified oligonucleotides. A single-stranded oligo with 42 bp can be utilised with the following composition: 5'-(A)25-(TC)8-T-N3-3' to create oligo-antibody conjugates (ssOligo-antibodies). In the oligo, the polyA sequence is hybridized with oligo(dT)25 pre-coated on the MBs, from which the coupling of the antibody to the MBs is performed. Intact EVs are released from the MBs, by cleavage of the hybridized oligo with DNase I (or generally, an enzyme, for example a nuclease, suitable for digesting/cleaving RNA or DNA strands). This method is suitable for 1-marker isolation of EVs captured with MBs (e.g., isolation of a population of EVs based only on targeting one surface marker of a target EV). This method can also be used for 2-marker isolations as generally described above, where Streptavidin-GNPs are bound to (target) EVs by a biotinylated antibody, attaching to a second marker on the EV. After DNase cleavage of the first marker, EV subpopulations can then be extracted using pH elution: For example: EV-bound GNPs are eluted (from each other) in an acidic buffer containing Glycine-HCl (pH 2.3). The acidic pH is neutralized and eluted EV subpopulations are available for downstream applications.

In a related example suitable for isolating EVs based on one EV marker, a complex/assembly comprised of MB-EVs-GNPs is dissolved in an acidic buffer containing Glycine-HCl (pH 2.3), resulting in eluted intact EVs and intact GNPs (e.g., the acidic conditions dissociate both the MBs and the GNPs from the EV). Eluted GNPs are washed, the acidic pH was neutralized and the GNPs are detected using the nanogaps as described above, from which the concentration of target EVs may be derived. As described above, the sizes of the MBs and GNPs are selected such that a one-to-one ratio of EVs to GNPs is formed in the MB-EVs-GNPs assemblies. Therefore, even after cleavage of a second linker linking the GNPs to target EVs, the number of GNPs present is representative of the number of target EVs captured.

As mentioned in the above disclosure, after isolating a set of EVs, it is possible to lyse said EVs in order to detect and characterise the internal contents/constituents of the EVs, such as cytosolic proteins. However, further methods allow for the detection of cytosolic EV markers without resorting to lysis. Such a method is permeabilization, which allows introduction of reporters in the EVs to detect biomolecules not positioned on an exterior EV surface. For example, permeabilization may performed with surfactants such as Saponin, Triton X-100 and others, that, when used, form complexes with cholesterol and/or other lipids in the EV membrane (or cell membranes) to generate pores, thus leaving internal content available for reporting

The above embodiments have been described by way of example only, and the described embodiments are to be considered in all respects only as illustrative and not restrictive. It will be appreciated that variations of the described embodiments may be made without departing from the scope of the invention.

## Claims

1. A method of isolating target extracellular vesicles, EVs, from a biofluid comprising a plurality of target EVs (106) and non-target EVs (102), the method comprising:

   introducing a plurality of EV capture microparticles (104) to the biofluid to obtain a precursor mixture, wherein the plurality of EV capture microparticles are functionalised, via a plurality of first linkers (402), with EV-specific binding agents specific to an EV surface marker, such that a plurality of bound microparticle-EV assemblies is formed in the precursor mixture;
   extracting the bound microparticle-EV assemblies to obtain a cleaned precursor mixture;
   introducing a plurality of target capture nanoparticles (116) to obtain an assembly mixture, wherein the plurality of target capture nanoparticles are functionalised, via a plurality of second linkers (404), with target-specific binding agents receptive to surface markers comprised on the target EVs, such that the target capture nanoparticles bind to the plurality of target EVs;
   cleaving the plurality of first linkers to dissociate at least the plurality of target EVs from the EV capture microparticles, wherein the plurality of second linkers are not cleaved;
   after the cleaving, extracting the plurality of EV capture microparticles and applying at least one of dielectrophoresis or centrifugation to extract the plurality of target capture nanoparticles, such that the target EVs are separated from the non-target EVs;
   after separating the plurality of target EVs from the non-target EVs, cleaving the plurality of second linkers to dissociate the plurality of target capture nanoparticles from the plurality of target EVs; and
   applying at least one of dielectrophoresis or centrifugation to extract the dissociated target capture nanoparticles and isolate a plurality of unbound target EVs.

2. A method as claimed in claim 1, wherein the plurality of second linkers comprise disulfide bonds, which are cleaved by treatment with a reducing or alkaline reagent.

3. A method as claimed in claim 1, wherein either:

the plurality of first linkers are photo-cleavable linkers, which are cleaved by exposure to UV or near-UV light; or
the plurality of first linkers comprise DNA or RNA (202), which are cleaved by treatment with an enzyme.

4. A method as claimed in any preceding claim, wherein at least one of the EV-specific binding agent or target-specific binding agent is an antibody, an antigen-binding fragment thereof or an aptamer.

5. A method as claimed in any preceding claim, further comprising removing unbound target capture nanoparticles before cleaving the plurality of first linkers.

6. A method as claimed in claim 1, wherein the plurality of first linkers comprise DNA or RNA, and the plurality of target capture nanoparticles comprise a plurality of second linkers comprising DNA or RNA (202), the method further comprising:
treating the assembly mixture with an enzyme to cleave a plurality of bound first linkers and a plurality of bound second linkers, the plurality of bound first and second linkers being bound to a target EV, to dissociate the plurality of target EVs from each of the EV capture microparticles and the target capture nanoparticles, such that the plurality of target EVs are separated from the plurality of non-target EVs; and optionally wherein the plurality of second linkers are terminated with:

a first receptor (108) bearing target antibodies which are receptive to target markers comprised on the target exosomes; and
a second receptor (112) bearing additional DNA or RNA which is receptive to the DNA or RNA in the plurality of first linkers.

7. A method as claimed in claim 6, further comprising:
applying at least one of dielectrophoresis or centrifugation to extract the dissociated target capture nanoparticles, to isolate a plurality of unbound target EVs.

8. A method as claimed in any one of claims 1 to 7, the method further comprising:

introducing the plurality of unbound target EVs to a second plurality of the EV capture microparticles;
introducing a second plurality of target capture nanoparticles functionalised with second target-specific binding agents receptive to surface markers comprised on a subset of the unbound target EVs;
cleaving the plurality of first linkers on the second plurality of the EV capture microparticles and extracting said second plurality of EV capture microparticles;
applying at least one of dielectrophoresis or centrifugation to extract the second plurality of target capture nanoparticles and isolate the subset of the target EVs; and
optionally wherein the method comprises:

analysing the plurality of unbound target EV, or when dependent on claim 10 the subset of the target EVs, to release an internal EV content; and
performing the characterisation on the internal EV content with one or more of: Polymerase Chain Reaction (PCR), Reverse Transcriptase-PCR, LC-MS, ELISA, SPR, DNA or RNA sequencing.

9. A method as claimed in any preceding claim, wherein the plurality of EV capture microparticles are magnetic, and wherein the steps of extracting the bound microparticle-EV assemblies and extracting the plurality of EV capture microparticles comprise applying a magnetic field.

10. A method as claimed in any one of claims 1 to 7, further comprising characterising the plurality of target EVs, the characterising comprising one or more of: Liquid chromatography-Mass Spectrometry (LC-MS), ELISA, Surface Plasmon Resonance (SPR), polymerase chain reaction (PCR), mass spectrometry, DNA or RNA sequencing, or liquid chromatography.

11. A method as claimed in claim 1, wherein each of the target EVs of the plurality of target EVs, having been separated from the non-target EVs, are comprised in a bound EV-nanoparticle assembly, the method further comprising:

applying an electric field between a pair of electrodes to concentrate the bound EV-nanoparticle assembly in a sensing region, wherein the sensing region is defined by a region between the pair of electrodes, which are separated by a lateral distance of less than 100 nm;
applying a nanoparticle sensing voltage between the electrodes;

characterizing a response of the sensing region to the nanoparticle sensing voltage to determine EV;

quantifying a number of target EVs from the quantification data.

12. A method as claimed in any preceding claim, wherein:

a length dimension of the plurality of EV capture microparticles is about an order of magnitude greater than a length dimension of each of the target EVs, such that, in the plurality of bound microparticle-EV assemblies, no two target EVs are proximate when bound to a surface of the plurality of EV capture microparticles.

13. A method as claimed in any preceding claim, wherein at least one of:

the plurality of target EVs have a size dimension of around 20 nm to 160 nm or around 50nm to 1 $\mu$m;

the plurality of target EVs are exosomes; or the plurality of target EVs are ectosomes.

14. A method as claimed in claim 1, comprising:

after separating the plurality of target EVs from the non-target EVs, introducing an acidic or oxidising reagent to dissociate the plurality of target capture nanoparticles from the respective plurality of target EVs, and optionally applying at least one of dielectrophoresis or centrifugation to extract the dissociated target capture nanoparticles and isolate a plurality of unbound target EVs.

15. A method as claimed in claim 13, wherein at least one of:

the plurality of EV capture microparticles are magnetic, and wherein the steps of extracting the bound microparticle-EV assemblies and extracting the plurality of EV capture microparticles comprise applying a magnetic field; or

the method further comprises removing unbound target- capture nanoparticles before the cleaving the plurality of first linkers, optionally wherein the biofluid is blood, urine, saliva, sputum, lymph cerebrospinal fluid, or is obtained from a cell culture.

**Patentansprüche**

1. Verfahren zum Isolieren von extrazellulären Ziel-Vesikeln, EVs, aus einem Biofluid, das eine Vielzahl von Ziel-EVs (106) und Nicht-Ziel-EVs (102) umfasst, wobei das Verfahren Folgendes umfasst:

Einbringen einer Vielzahl von EV-Fang-Mikropartikeln (104) in das Biofluid, um eine Vorläufermischung zu erhalten, wobei die Vielzahl von EV-Fang-Mikropartikeln über eine Vielzahl von ersten Linkern (402) mit EV-spezifischen Bindemitteln funktionalisiert sind, die für einen EV-Oberflächenmarker spezifisch sind, sodass eine Vielzahl von gebundenen Mikropartikel-EV-Anordnungen in der Vorläufermischung gebildet werden;

Extrahieren der gebundenen Mikropartikel-EV-Anordnungen, um eine gereinigte Vorläufermischung zu erhalten,

Einbringen einer Vielzahl von Ziel-Fang-Nanopartikeln (116), um eine Anordnungsmischung zu erhalten, wobei die Vielzahl von Ziel-Fang-Nanopartikeln über eine Vielzahl von zweiten Linkern (404) mit zielspezifischen Bindemitteln funktionalisiert sind, die für Oberflächenmarker, die auf den Ziel-EVs enthalten sind, aufnahmefähig sind, sodass die Ziel-Fang-Nanopartikel an die Vielzahl von Ziel-EVs binden;

Spalten der Vielzahl von ersten Linkern, um mindestens die Vielzahl von Ziel-EVs von den EV-Fang-Mikropartikeln zu dissoziieren, wobei die Vielzahl von zweiten Linkern nicht gespalten werden;

nach dem Spalten, Extrahieren der Vielzahl von EV-Fang-Mikropartikeln und Anwenden von mindestens einer von Dielektrophorese oder Zentrifugation, um die Vielzahl von Ziel-Fang-Nanopartikeln zu extrahieren, sodass die Ziel-EVs von den Nicht-Ziel-EVs getrennt werden;

nach dem Trennen der Vielzahl von Ziel-EVs von den Nicht-Ziel-EVs, Spalten der Vielzahl von zweiten Linkern, um die Vielzahl von Ziel-Fang-Nanopartikeln von der Vielzahl von Ziel-EVs zu dissoziieren, und

Anwenden mindestens einer von Dielektrophorese oder Zentrifugation, um die dissoziierten Ziel-Fang-Nanopartikel zu extrahieren und eine Vielzahl von ungebundenen Ziel-EVs zu isolieren.

2. Verfahren nach Anspruch 1, wobei die Vielzahl von zweiten Linkern Disulfidbindungen umfassen, die durch Behandlung mit einem reduzierenden oder alkalischen Reagens gespalten sind.

3. Verfahren nach Anspruch 1, wobei entweder:

die Vielzahl von ersten Linkern photospaltbare Linker sind, die durch Aussetzung gegenüber UV- oder UV-nahem Licht gespalten werden; oder

die Vielzahl von ersten Linkern DNA oder RNA (202) umfassen, die durch Behandlung mit einem Enzym gespalten werden.

4. Verfahren nach einem der vorhergehenden Ansprüche, wobei mindestens eines von dem EV-spezifischen Bindemittel oder dem zielspezifischen Bindemittel ein Antikörper, ein antigenbindendes Fragment davon oder ein Aptamer ist.

5. Verfahren nach einem der vorhergehenden Ansprüche, ferner umfassend das Entfernen von ungebundenen Ziel-Fang-Nanopartikeln vor dem Spalten der Vielzahl von ersten Linkern.

6. Verfahren nach Anspruch 1, wobei die Vielzahl von ersten Linkern DNA oder RNA umfassen, und die Vielzahl von Ziel-Fang-Nanopartikeln eine Vielzahl von zweiten Linkern umfassend DNA oder RNA (202) umfassen, wobei das Verfahren ferner Folgendes umfasst:

Behandeln der Anordnungsmischung mit einem Enzym, um eine Vielzahl von gebundenen ersten Linkern und eine Vielzahl von gebundenen zweiten Linkern zu spalten, wobei die Vielzahl von gebundenen ersten und zweiten Linkern an ein Ziel-EV gebunden sind, um die Vielzahl von Ziel-EVs von jedem der EV-Fang-Mikropartikel und der Ziel-Fang-Mikropartikel zu dissoziieren, sodass die Vielzahl von Ziel-EVs von der Vielzahl von Nicht-Ziel-EVs getrennt werden; und optional wobei die Vielzahl von zweiten Linkern mit Folgendem abgeschlossen werden:

einem ersten Rezeptor (108), der Ziel-Antikörper trägt, die für Zielmarker, die auf den Zielexosomen umfasst sind, aufnahmefähig sind, und einem zweiten Rezeptor (112), der zusätzliche DNA oder RNA trägt, die für die DNA oder RNA in der Vielzahl von ersten Linkern aufnahmefähig ist.

7. Verfahren nach Anspruch 6, ferner Folgendes umfassend:
Anwenden mindestens einer von Dielektrophorese oder Zentrifugation, um die dissoziierten Ziel-Fang-Nanopartikel zu extrahieren, um eine Vielzahl von ungebundenen Ziel-EVs zu isolieren.

8. Verfahren nach einem der Ansprüche 1 bis 7, wobei das Verfahren ferner Folgendes umfasst:

Einbringen der Vielzahl von ungebunden Ziel-EVs zu einer zweiten Vielzahl der EV-Fang-Mikropartikel;
Einbringen einer zweiten Vielzahl von Ziel-Fang-Nanopartikeln, die mit zweiten zielspezifischen Bindemitteln funktionalisiert sind, die für Oberflächenmarker, die auf einer Teilmenge der ungebundenen Ziel-EVs enthalten sind, aufnahmefähig sind,

Spalten der Vielzahl von ersten Linkern auf der zweiten Vielzahl der EV-Fang-Mikropartikel und Extrahieren der zweiten Vielzahl von EV-Fang-Mikropartikeln;
Anwenden mindestens einer von Dielektrophorese oder Zentrifugation, um die zweite Vielzahl von Ziel-Fang-Nanopartikeln zu extrahieren und die Teilmenge der Ziel-EVs zu isolieren; und optional wobei das Verfahren Folgendes umfasst:

Analysieren der Vielzahl ungebundener Ziel-EVs oder, in Abhängigkeit von Anspruch 10 betrachtet, der Teilmenge der Ziel-EVs, um einen internen EV-Gehalt freizusetzen; und
Durchführen der Kennzeichnung auf dem internen EV-Gehalt mit einer oder mehreren von Folgenden: Polymerase-Kettenreaktion (PCR), Reverse-Transkriptase-PCR, LC-MS, ELISA, SPR, DNA- oder RNA-Sequenzierung.

9. Verfahren nach einem der vorhergehenden Ansprüche, wobei die Vielzahl von EV-Fang-Mikropartikeln magnetisch sind, und wobei die Schritte des Extrahierens der gebundenen Mikropartikel-EV-Anordnungen und des Extrahierens der Vielzahl von EV-Fang-Mikropartikeln das Anlegen eines Magnetfelds umfassen.

10. Verfahren nach einem der Ansprüche 1 bis 7, ferner umfassend das Kennzeichnen der Vielzahl von Ziel-EVs, wobei das Kennzeichnen eine oder mehrere von Folgenden umfasst: Flüssigchromatographie-Massenspektrometrie (LC-MS), ELISA, Oberflächenplasmonenresonanz (SPR), Polymerase-Kettenreaktion (PCR), Massenspektrometrie, DNA- oder RNA-Sequenzierung, oder Flüssigchromatographie.

11. Verfahren nach Anspruch 1, wobei jedes der Ziel-EVs aus der Vielzahl von Ziel-EVs, die von den Nicht-Ziel-EVs getrennt wurden, in einer gebundenen EV-Nanopartikel-Anordnung umfasst ist, wobei das Verfahren ferner Folgendes umfasst:

Anlegen eines elektrischen Feldes zwischen einem Paar von Elektroden, um die gebundene EV-Nanopartikel-Anordnung in einem Erfassungsbereich zu konzentrieren, wobei der Erfassungsbereich durch einen Bereich zwischen dem Paar von Elektroden definiert ist, die durch eine seitliche Entfernung von weniger als 100 nm getrennt sind;
Anlegen einer Nanopartikel-Erfassungsspannung zwischen den Elektroden;
Kennzeichnen eines Ansprechens des Erfassungsbereichs auf die Nanopartikel-Erfassungsspannung, um EVs zu bestimmen; Quantifizieren einer Anzahl von Ziel-EVs aus den Quantifizierungsdaten.

12. Verfahren nach einem der vorhergehenden Ansprüche, wobei:
eine Längenabmessung der Vielzahl von EV-Fang-Mikropartikeln ungefähr eine Größenordnung größer ist als eine Längenabmessung jedes der Ziel-EVs, sodass in der Vielzahl von gebundenen Mikropartikel-EV-Anordnungen keine zwei Ziel-EVs benachbart sind, wenn sie an eine Oberfläche der Vielzahl von EV-Fang-Mikropartikeln gebunden sind.

13. Verfahren nach einem der vorhergehenden Ansprüche, wobei mindestens eines von Folgendem zutrifft:

die Vielzahl von Ziel-EVs weisen eine Größenabmessung von ungefähr 20 nm bis 160 nm oder ungefähr 50 nm bis 1 μm auf;
die Vielzahl von Ziel-EVs sind Exosomen; oder
die Vielzahl von Ziel-EVs sind Ektosomen.

14. Verfahren nach Anspruch 1, Folgendes umfassend:
nach dem Trennen der Vielzahl von Ziel-EVs von den Nicht-Ziel-EVs, Einbringen eines sauren oder oxidierenden Reagens, um die Vielzahl von Ziel-Fang-Nanopartikeln von der jeweiligen Vielzahl von Ziel-EVs zu dissoziieren, und optional Anwenden mindestens einer von Dielektrophorese oder Zentrifugation, um die dissoziierten Ziel-Fang-Nanopartikel zu extrahieren und eine Vielzahl von ungebundenen Ziel-EVs zu isolieren.

15. Verfahren nach Anspruch 13, wobei mindestens eines von Folgendem zutrifft:

die Vielzahl von EV-Fang-Mikropartikeln sind magnetisch, und wobei die Schritte des Extrahierens der gebundenen Mikropartikel-EV-Anordnungen und des Extrahierens der Vielzahl von EV-Fang-Mikropartikeln das Anlegen eines Magnetfelds umfassen; oder
das Verfahren umfasst ferner das Entfernen ungebundener Ziel-Fang-Nanopartikel vor dem Spalten der Vielzahl von ersten Linkern, wobei optional das Biofluid Blut, Urin, Speichel, Sputum, lymphatisches Liquor cerebrospinalis ist oder aus einer Zellkultur erhalten wird.

**Revendications**

1. Procédé d'isolation de vésicules extra-cellulaires cibles, EV, d'un biofluide comprenant une pluralité d'EV cibles (106) et d'EV non-cibles (102), le procédé comprenant :

l'introduction d'une pluralité de microparticules de capture d'EV (104) dans le biofluide pour obtenir un mélange précurseur, dans lequel la pluralité de microparticules de capture d'EV sont fonctionnalisées, par le biais d'une pluralité de premiers lieurs (402), avec des agents de liaison spécifiques à des EV, spécifiques à un marqueur de surface d'EV, de sorte qu'une pluralité d'ensembles EV-microparticules liés se forment dans le mélange précurseur ;
l'extraction des ensembles EV-microparticules liés pour obtenir un mélange précurseur nettoyé ;
l'introduction d'une pluralité de nanoparticules de capture cibles (116) pour obtenir un mélange d'assemblage, dans lequel la pluralité de nanoparticules de capture cibles sont fonctionnalisées, par le biais d'une pluralité de deuxièmes lieurs (404), avec des agents de liaison spécifiques à la cible réceptifs à des marqueurs de surface compris sur les EV cibles, de sorte que les nanoparticules de capture cibles se lient à la pluralité d'EV cibles ;
le clivage de la pluralité de premiers lieurs pour dissocier au moins la pluralité d'EV cibles des microparticules de capture d'EV, dans lequel la pluralité de deuxièmes lieurs ne sont pas clivés ;
après le clivage, l'extraction de la pluralité de microparticules de capture d'EV et l'application d'au moins une parmi une diélectrophorèse ou une centrifugation pour extraire la pluralité de nanoparticules de capture cibles, de sorte que les EV cibles sont séparées des EV non-cibles ;
après séparation de la pluralité d'EV cibles des EV non-cibles, le clivage de la pluralité de deuxièmes lieurs pour dissocier la pluralité de nanoparticules de capture cibles de la pluralité d'EV cibles ; et
l'application d'au moins une parmi une diélectrophorèse ou une centrifugation pour extraire les nanoparticules de capture cibles dissociées et isoler une pluralité d'EV cibles non liées.

2. Procédé selon la revendication 1, dans lequel la pluralité de deuxièmes lieurs comprennent des liaisons disulfure, qui sont clivées par traitement avec un agent réducteur ou alcalin.

3. Procédé selon la revendication 1, dans lequel soit :

la pluralité de premiers lieurs sont des lieurs photo-clivables, qui sont clivés par exposition à une lumière UV ou une lumière proche UV ; soit la pluralité de premiers lieurs comprend de l'ADN ou de l'ARN (202), qui sont clivés par traitement avec une enzyme.

4. Procédé selon l'une quelconque des revendications précédentes, dans lequel au moins un de l'agent de liaison spécifique à des EV ou de l'agent de liaison spécifique à la cible est un anticorps, un fragment de liaison à un antigène de celui-ci ou un aptamère.

5. Procédé selon l'une quelconque des revendications précédentes, comprenant en outre l'enlèvement de nanoparticules de capture cible non liées avant le clivage de la pluralité de premiers lieurs.

6. Procédé selon la revendication 1, dans lequel la pluralité de premiers lieurs comprend de l'ADN ou de l'ARN, et la pluralité de nanoparticules de capture cibles comprennent une pluralité de deuxièmes lieurs comprenant de l'ADN ou de l'ARN (202), le procédé comprenant en outre :

le traitement du mélange d'ensemble avec une enzyme pour cliver une pluralité de premiers lieurs liés et une pluralité de deuxièmes lieurs liés, la pluralité de premiers et deuxièmes lieurs liés étant liés à une EV cible, pour dissocier la pluralité d'EV cibles de chacune des microparticules de capture d'EV et des nanoparticules de capture cibles, de sorte que la pluralité d'EV cibles soient séparées de la pluralité d'EV non-cibles ;
et facultativement dans lequel la pluralité de deuxièmes lieurs se terminent par :

un premier récepteur (108) portant des anticorps cibles qui sont réceptifs à des marqueurs cibles compris sur les exosomes cibles ; et
un deuxième récepteur (112) portant de l'ADN ou de l'ARN supplémentaire qui est réceptif à l'ADN ou à l'ARN dans la pluralité des premiers lieurs.

7. Procédé selon la revendication 6, comprenant en outre :
l'application d'au moins une parmi une diélectrophorèse ou une centrifugation pour extraire les nanoparticules de capture cibles dissociées, pour isoler une pluralité d'EV cibles non liées.

8. Procédé selon l'une quelconque des revendications 1 à 7, le procédé comprenant en outre :

l'introduction de la pluralité d'EV cibles non liées à une deuxième pluralité des microparticules de capture d'EV ;
l'introduction d'une deuxième pluralité de nanoparticules de capture cibles fonctionnalisées avec des deuxièmes agents de liaison spécifiques à la cible réceptifs à des marqueurs de surface compris sur un sous-ensemble des EV cibles non liées ;

le clivage de la pluralité de premiers lieurs sur la deuxième pluralité des microparticules de capture d'EV et l'extraction de ladite deuxième pluralité de microparticules de capture d'EV ;
l'application d'au moins une parmi une diélectrophorèse ou une centrifugation pour extraire la deuxième pluralité de nanoparticules de capture cibles et isoler le sous-ensemble des EV cibles ; et
facultativement dans lequel le procédé comprend :

l'analyse de la pluralité d'EV cibles non liées, ou lorsqu'elle dépend de la revendication 10, du sous-ensemble des EV cibles, pour libérer un contenu d'EV interne ; et
la réalisation de la caractérisation sur le contenu d'EV interne avec une ou plusieurs parmi : la Réaction en Chaîne par Polymérase (PCR), la PCR à transcriptase inverse, LC-MS, ELISA, SPR, un séquençage d'ADN ou d'ARN.

9. Procédé selon l'une quelconque des revendications précédentes, dans lequel la pluralité de microparticules de capture d'EV sont magnétiques, et dans lequel les étapes consistant à extraire les ensembles d'EV-microparticules liés, et à extraire la pluralité de microparticules de capture d'EV comprennent l'application d'un champ magnétique.

10. Procédé selon l'une quelconque des revendications 1 à 7, comprenant en outre la caractérisation de la pluralité d'EV cibles, la caractérisation comprenant une ou plusieurs parmi : la chromatographie en phase liquide-spectrométrie de masse (LC-MS), ELISA, la Résonance Plasmon de Surface (SPR), la réaction en chaîne par polymérase (PCR), la spectrométrie de masse, le séquençage d'ADN ou d'ARN, ou la chromatographie en phase liquide.

11. Procédé selon la revendication 1, dans lequel chacune des EV cibles de la pluralité d'EV cibles, ayant été séparées des EV non-cibles, est comprise dans un ensemble de nanoparticules-EV liées, le procédé comprenant en outre :

l'application d'un champ électrique entre une paire d'électrodes pour concentrer l'ensemble EV-nanoparticules liées dans une région de détection, dans lequel la région de détection est définie par une région entre la paire d'électrodes, qui sont séparées par une distance latérale inférieure à 100 nm ;
l'application d'une tension de détection de nanoparticules entre les électrodes ;
la caractérisation d'une réponse de la région de détection à la tension de détection de nanoparticules pour déterminer des EV ;
la quantification d'un nombre d'EV cibles à partir des données de quantification.

**12.** Procédé selon l'une quelconque des revendications précédentes, dans lequel :
une dimension de la longueur de la pluralité de microparticules de capture d'EV est environ d'un ordre d'amplitude supérieur à une dimension de la longueur de chacune des EV cibles, de sorte que, dans la pluralité d'ensembles EV-microparticules liées, aucune paire d'EV cibles n'est proche lorsqu'elles sont liées à une surface de la pluralité de microparticules de capture d'EV.

**13.** Procédé selon l'une quelconque des revendications précédentes, dans lequel au moins une parmi les propositions suivantes s'applique :

la pluralité d'EV cibles présentent une taille d'environ 20 nm à 160 nm ou d'environ 50 nm à 1 $\mu$m ;
la pluralité d'EV cibles sont des exosomes ; ou la pluralité d'EV cibles sont des ectosomes.

**14.** Procédé selon la revendication 1, comprenant :
après la séparation de la pluralité d'EV cibles des EV non-cibles, l'introduction d'un réactif acide ou oxydant pour dissocier la pluralité de nanoparticules de capture cibles de la pluralité respective d'EV cibles, et facultativement l'application d'au moins une parmi une diélectrophorèse ou une centrifugation pour extraire les nanoparticules de capture cibles dissociées et isoler une pluralité d'EV cibles non liées.

**15.** Procédé selon la revendication 13, dans lequel au moins une des propositions suivantes s'applique :

la pluralité de microparticules de capture d'EV sont magnétiques, et dans lequel les étapes consistant à extraire les ensembles EV-microparticules liées et à extraire la pluralité de microparticules de capture d'EV comprennent l'application d'un champ magnétique ; ou
le procédé comprend en outre l'enlèvement de nanoparticules de capture cibles non liées avant le clivage de la pluralité de premiers lieurs, facultativement dans lequel le biofluide est du sang, de l'urine, de la salive, des crachats, du liquide céphalo-rachidien lymphatique, ou est obtenu à partir d'une culture cellulaire.

Figure 1a

Figure 1b

Figure 1c

Figure 1d

Figure 1e

Figure 2a

Figure 2b

Figure 2c

Figure 2d

Figure 2e

EP 4 172 589 B1

Figure 3a

Figure 3b

Figure 3c

Figure 3d

Figure 3e

Figure 4a

Figure 4b

Figure 4c

Figure 5b

Figure 5a

**Figure 6**

**Figure 7**

Figure 8

Figure 9

S100: Receive biofluid and (optionally) remove larger contaminants based on size to isolate a population of extracellular vesicles (EVs) or viruses

S102: Attach population of EVs/viruses to a magnetic microparticle

S103: Extract magnetic microparticle attached to EVs/viruses from the mixture with magnetic field to obtain cleaned mixture free of contaminants

S104: Tag a target population of EVs/Viruses with GNPs

S106: Extract magnetic microparticle attached to EVs/viruses from the mixture with magnetic field to remove unbound GNPs

S108: Cleave magnetic microparticle from captured EVs/viruses

S110: Extract magnetic microparticle with magnetic field

S112: Separate GNP-tagged target EVs/viruses to isolate target EV population from non-target particles

S114: **Quantification** - Attract target EVs bound to GNPs to nanogap electrodes for quantification and sensing

S116: **Characterisation** - Remove GNP tag and characterise contents/surface of target EVs or target viruses

Figure 10

S200: Isolate target (extracellular vesicles or viruses) bound to GNP

S202: Quantification and sensing using nanogap electrode array

S204: Cleave target from GNP and extract target

S206: Lyse target to open membrane (optional)

S208: Surface protein and cytosolic protein discovery via liquid chromatography, Mass spectrometry, ELISA etc.

S210: RNA and/or DNA discovery via RNA sequencing (e.g. PCR and sequencing)

Figure 11

BIOFLUID —1200

TARGET PARTICLE
NON-TARGET PARTICLES
MAGNETIC MICROPARTICLES
CONTAMINANTS —1202

CONTAMINANTS —1203

TARGET PARTICLE
NON-TARGET PARTICLES
MAGNETIC MICROPARTICLES
TARGET CAPTURE NANOPARTICLES —1204

1205
UNBOUND TARGET
CAPTURE
NANOPARTICLES

TARGET ASSEMBLIES
CLEAVING AGENT/TREATMENT —1206

MAGNETIC
MICROPARTICLES —1207

NANOPARTICLES BOUND TO TARGETS
NON-TARGET PARTICLES —1208

ISOLATED TARGETS —1210

Figure 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20190025330 A
- US 2019025330 A1

- WO 2019211622 A1

**Non-patent literature cited in the description**

- **AN.** Magneto-mediated electrochemical sensor... *Analytical Chemistry,* April 2020, vol. 92, 5404-5410

- **BORIACHEK.** Quantum dot-based sensitive detection... *The Analyst,* 2017, vol. 142, 2211-2219